(19) Europäisches Patentamt
European Patent Office
Office européen des brevets

(11) **EP 4 039 279 A1**

(12) # EUROPEAN PATENT APPLICATION
published in accordance with Art. 153(4) EPC

(43) Date of publication:
**10.08.2022 Bulletin 2022/32**

(21) Application number: **20871804.9**

(22) Date of filing: **02.10.2020**

(51) International Patent Classification (IPC):
***A61K 47/61*** (2017.01) ***A61P 13/10*** (2006.01)
***A61P 43/00*** (2006.01) ***A61K 45/00*** (2006.01)
***G01N 33/15*** (2006.01) ***G01N 33/50*** (2006.01)
***A61K 31/192*** (2006.01) ***A61K 31/196*** (2006.01)
***A61K 31/495*** (2006.01) ***A61K 31/575*** (2006.01)
***A61K 31/58*** (2006.01)

(52) Cooperative Patent Classification (CPC):
**A61K 31/192; A61K 31/196; A61K 31/495; A61K 31/575; A61K 31/58; A61K 45/00; A61K 47/61; A61P 13/10; A61P 43/00; G01N 33/15; G01N 33/50**

(86) International application number:
**PCT/JP2020/037640**

(87) International publication number:
**WO 2021/066175 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.10.2019 JP 2019183082**

(71) Applicant: **Seikagaku Corporation**
**Tokyo 100-0005 (JP)**

(72) Inventors:
- **NARITA, Muneto**
  **Tokyo 100-0005 (JP)**
- **ISHII, Kensuke**
  **Tokyo 100-0005 (JP)**
- **SHIMURA, Akihito**
  **Tokyo 100-0005 (JP)**

(74) Representative: **Hoffmann Eitle**
**Patent- und Rechtsanwälte PartmbB**
**Arabellastraße 30**
**81925 München (DE)**

(54) **TRANSMUCOSAL DELIVERY SYSTEM FOR MEDICINAL ACTIVE INGREDIENT TO SUBMUCOSA OF BLADDER**

(57) As a system that enables effective delivery of a pharmaceutical active ingredient to a submucosal tissue of the bladder, a transmucosal delivery system including a conjugate of a hydrophobic compound containing the pharmaceutical active ingredient and chondroitin sulfate is provided.

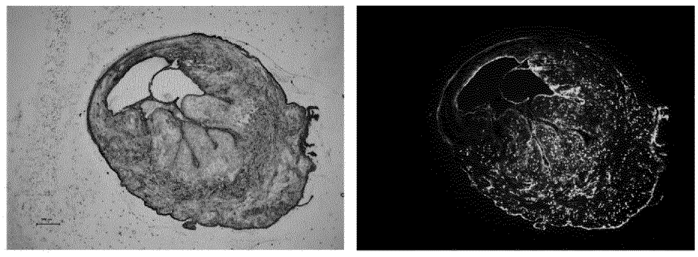

(a) (b)

FIG. 1

EP 4 039 279 A1

## Description

Technical Field

The present invention relates to a transmucosal delivery system for a pharmaceutical active ingredient to a submucosal tissue of the bladder, a method for screening a drug candidate compound for treating a bladder disease, and a method for giving drug efficacy to a submucosal tissue of the bladder.

Background Art

Efforts for the treatment of a bladder disease utilizing a glycosaminoglycan have been made. For example, a case where a chondroitin sulfate derivative in which a group derived from a steroid is bound to a group derived from chondroitin sulfate is used as a bladder disease treatment agent has been known (For example, Patent Literature 1). In addition, there has also been a report that chondroitin sulfate itself can be used for interstitial cystitis (IC) that is a bladder disease as a therapeutic agent (For example, Non Patent Literature 1). Further, Patent Literature 2 describes an invention relating to a derivative of hyaluronic acid that is one type of glycosaminoglycan as a compound showing high retentivity in a mucosal epithelial layer, and a bladder mucosa is exemplified as one of the mucosae.

In some of the above-mentioned prior arts, a description that a bladder disease treatment agent injected into the bladder reaches and adheres to the bladder wall, or the like is also found. However, in any of the above-mentioned prior arts, it is not disclosed or suggested that a pharmaceutical active ingredient administered to the lumen of the bladder is delivered to a submucosal tissue of the bladder. That is, it cannot be said that a method for effectively delivering a pharmaceutical active ingredient to an inflammation or the like occurring in a submucosal tissue of the bladder has been established.

The present invention has been conducted for solving the problem as described above, and has its object to provide a system that enables effective delivery of a pharmaceutical active ingredient administered to the lumen of the bladder to a submucosal tissue.

Citation List

Patent Literature

Patent Literature 1: WO 2016/111356
Patent Literature 2: WO 2007/043702

Non Patent Literature

Non Patent Literature 1: Paul J. Hauser, and other 5 persons, "Restoring Barrier Function to Acid-Damaged Bladder by Intravesical Chondroitin Sulfate", The Journal of Urology, Vol. 182, pp. 2477-2482, 2009

Summary of Invention

The present inventors conducted intensive studies for achieving the above object, and as a result, they found that a pharmaceutical active ingredient derivatized by chondroitin sulfate is effective in achieving effective delivery of the pharmaceutical active ingredient to a submucosal tissue of the bladder, and thus completed the present invention.

That is, the present invention is directed to a transmucosal delivery system for a pharmaceutical active ingredient to a submucosal tissue of the bladder, including a conjugate of chondroitin sulfate and a hydrophobic compound (hereinafter the conjugate is also referred to as "chondroitin sulfate derivative"), wherein the hydrophobic compound contains the pharmaceutical active ingredient.

Brief Description of Drawings

[FIG. 1] FIG. 1 shows a bright field image (a) of a frozen section on day 1 after administering a test substance using a fluorescently labeled compound of Example 1, and a fluorescent image (b) corresponding thereto.
[FIG. 2] FIG. 2 shows a bright field image (a) of a frozen section on day 1 after administering a test substance using

a fluorescently labeled compound of Example 2, and a fluorescent image (b) corresponding thereto.

[FIG. 3] FIG. 3 shows a bright field image (a) of a frozen section on day 1 after administering a test substance using a fluorescently labeled compound of Example 3, and a fluorescent image (b) corresponding thereto.

[FIG. 4] FIG. 4 shows a bright field image (a) of a frozen section on day 1 after administering a test substance using a fluorescently labeled compound of Example 4, and a fluorescent image (b) corresponding thereto.

[FIG. 5] FIG. 5 shows a bright field image (a) of a frozen section on day 1 after administering a test substance using a fluorescently labeled compound of Example 5, and a fluorescent image (b) corresponding thereto.

[FIG. 6] FIG. 6 shows a bright field image (a) of a frozen section on day 1 after administering a test substance using a fluorescently labeled compound of Example 6, and a fluorescent image (b) corresponding thereto.

[FIG. 7] FIG. 7 shows a bright field image (a) of a frozen section on day 1 after administering a test substance using a fluorescently labeled compound of Comparative Example 1, and a fluorescent image (b) corresponding thereto.

[FIG. 8] FIG. 8 shows a bright field image (a) of a frozen section on day 1 after administering a test substance using a fluorescently labeled compound of Comparative Example 2, and a fluorescent image (b) corresponding thereto.

[FIG. 9] FIG. 9 shows a bright field image (a) of a frozen section on day 1 after administering a test substance using a fluorescently labeled compound of Comparative Example 3, and a fluorescent image (b) corresponding thereto.

Description of Embodiments

According to the present invention, a delivery system that enables effective delivery of a pharmaceutical active ingredient administered to the lumen of the bladder to a submucosal tissue, and various methods associated with the delivery system can be provided. It is known that the permeation of a transmucosal compound increases as the molecular weight of the compound decreases. The present invention is at least partially based on the surprising finding that when a conjugate of chondroitin sulfate that is a polymer and a pharmaceutical active ingredient is administered to the lumen of the bladder, more effective delivery to a submucosal tissue can be achieved as compared with a case where the pharmaceutical active ingredient itself is administered as a single agent.

The term "step" in the present description not only includes an independent step, but also includes a step even if the step cannot be clearly distinguished from another step as long as the intended object of the step is achieved. Further, a numerical value range indicated using "to" indicates a range that includes the numerical values described before and after the "to" as the minimum value and the maximum value, respectively. Further, when a plurality of substances corresponding to each component are present in a composition, the content of the component in the composition means the total amount of the plurality of substances present in the composition unless otherwise stated.

Hereinafter, embodiments of the present invention will be described in detail. The present invention is not limited to the following embodiments, and it should be understood that those obtained by appropriately adding a change, a modification, or the like to the following embodiments based on a common knowledge of a person skilled in the art are included in the scope of the present invention without departing from the gist of the present invention.

(First Embodiment)

A first embodiment of the present invention relates to, in one aspect, a transmucosal delivery system for a pharmaceutical active ingredient to a submucosal tissue of the bladder. That is, in the first embodiment of the present invention, a transmucosal delivery system for a pharmaceutical active ingredient to a submucosal tissue of the bladder, including a conjugate of chondroitin sulfate and a hydrophobic compound, wherein the hydrophobic compound contains the pharmaceutical active ingredient is provided.

The first embodiment of the present invention relates to, in another aspect, a targeted system for a pharmaceutical active ingredient to a submucosal tissue of the bladder. That is, in the first embodiment of the present invention, a targeted system for a pharmaceutical active ingredient to a submucosal tissue of the bladder, including a conjugate of chondroitin sulfate and a hydrophobic compound, wherein the hydrophobic compound contains the pharmaceutical active ingredient is provided.

The first embodiment of the present invention relates to, in still another aspect, a method for transmucosally delivering a pharmaceutical active ingredient to a submucosal tissue of the bladder. The method includes a step of administering a conjugate of chondroitin sulfate and a hydrophobic compound to the lumen of the bladder of a subject. That is, in the first embodiment of the present invention, a method for transmucosally delivering a pharmaceutical active ingredient to a submucosal tissue of the bladder, including a step of administering a conjugate of chondroitin sulfate and a hydrophobic compound to the lumen of the bladder of a subject, wherein the hydrophobic compound contains the pharmaceutical active ingredient is also provided.

The first embodiment of the present invention relates to, in still another aspect, a conjugate for use in transmucosal delivery of a pharmaceutical active ingredient to a submucosal tissue of the bladder. That is, in the first embodiment of the present invention, a conjugate for use in transmucosal delivery of a pharmaceutical active ingredient to

a submucosal tissue of the bladder, wherein the conjugate is a conjugate of chondroitin sulfate and a hydrophobic compound, and the hydrophobic compound contains the pharmaceutical active ingredient is also provided.

The first embodiment of the present invention relates to, in still another aspect, a method for transmucosally having a pharmaceutical active ingredient permeate into a submucosal tissue of the bladder and releasing the pharmaceutical active ingredient in a sustained manner in the submucosal tissue by binding a hydrophobic compound containing the pharmaceutical active ingredient to chondroitin sulfate. That is, in the first embodiment of the present invention, a method for transmucosally delivering (permeating) a pharmaceutical active ingredient to a submucosal tissue of the bladder and releasing the pharmaceutical active ingredient in a sustained manner in the submucosal tissue by binding a hydrophobic compound containing the pharmaceutical active ingredient to chondroitin sulfate, including a step of administering a conjugate of chondroitin sulfate and the hydrophobic compound to the lumen of the bladder of a subject is also provided.

The first embodiment of the present invention relates to, in still another aspect, a prodrug of a pharmaceutical active ingredient. That is, in the first embodiment of the present invention, a prodrug of a pharmaceutical active ingredient, wherein the prodrug is a conjugate of chondroitin sulfate and a hydrophobic compound, the hydrophobic compound releasably contains the pharmaceutical active ingredient, and the prodrug is used for transmucosally delivering the pharmaceutical active ingredient to a submucosal tissue of the bladder is also provided.

(1) Chondroitin Sulfate Derivative (also referred to as "Conjugate")

The chondroitin sulfate derivative has a structure in which chondroitin sulfate (hereinafter sometimes abbreviated as "CS") and a hydrophobic compound are bound to each other. Further, the hydrophobic compound contains a pharmaceutical active ingredient (hereinafter sometimes abbreviated as "API"). Here, the pharmaceutical active ingredient refers to an arbitrary type of pharmaceutically active compound useful for prevention, diagnosis, stabilization, treatment, or the like of a condition, a disorder, or a disease. Preferably, the pharmaceutical active ingredient is a compound to be used for the purpose of treating a disease. In an embodiment, API can be a compound that has received Pharmaceutical Affairs regulatory approval from the Bureau of Pharmaceutical Affairs in any country or district such as the United States Food and Drug Administration or the Ministry of Health, Labour and Welfare in Japan.

The chondroitin sulfate derivative makes it easy to effectively deliver API to a submucosal tissue of the bladder when it is administered to the lumen of the bladder of a subject. In an embodiment, the chondroitin sulfate derivative can be a prodrug of API. Typically, the prodrug is a compound in which API is metabolized or converted into an active form or a highly active form after administration.

CS is present as a group derived from CS in the chondroitin sulfate derivative. Therefore, the chondroitin sulfate derivative contains at least one type of group derived from CS. In an embodiment, the group derived from CS is a group formed by removing a hydroxy group from a carboxy group contained in CS. In the chondroitin sulfate derivative, the group derived from CS can correspond to a carrier.

CS is not particularly limited as long as it has a structure in which a sulfate group is held in a glycan backbone formed by repeating two sugars of D-glucuronic acid (or L-iduronic acid) and N-acetyl-D-galactosamine in a two-sugar unit. Further, an acidic functional group including a carboxy group and a sulfate group contained in CS may be in a free state where a salt is not formed or in a state where a pharmaceutically acceptable salt is formed.

Examples of the pharmaceutically acceptable salt include salts with an alkali metal ion such as a sodium salt and a potassium salt, and salts with an alkaline earth metal ion such as a magnesium salt and a calcium salt. CS is preferably a salt with a pharmaceutically acceptable alkali metal ion and more preferably a sodium salt from the viewpoint of applicability to a living body and affinity therefor.

CS can be produced by a known method according to the type thereof. As a method for producing CS, for example, extraction and purification from an animal derived raw material, culture and purification from a chondroitin-producing bacterium or the like, modification with glycan, glycan synthesis, and the like can be exemplified.

The weight average molecular weight of CS is not particularly limited, and can be appropriately selected according to the intended purpose or the like. The weight average molecular weight of CS is, for example, 4,000 or more, preferably from 6,000 to 300,000, and more preferably from 10,000 to 100,000. The weight average molecular weight of CS can be measured by a light scattering method.

In an embodiment, the weight average molecular weight of CS is 10 times or more the molecular weight of API.

API is present as a group derived from API in the chondroitin sulfate derivative. Therefore, the chondroitin sulfate derivative contains at least one type of group derived from API. In an embodiment, the group derived from API is a group formed by removing a hydrogen atom from a hydroxy group or a hydroxy group from a carboxy group contained in API. Note that the position of the hydroxy group from which a hydrogen atom is removed is not particularly limited. In the chondroitin sulfate derivative, the group derived from API is preferably linked to a group derived from CS or the below-mentioned spacer through a covalent bond that is degradable in a living body (for example, a covalent bond such as an ester bond, a thioester bond, or an amide bond that is degradable by hydrolysis or an in vivo enzyme such as a

carboxylesterase).

As an example of API, although not particularly limited, a compound to be used for the purpose of treating a bladder disease is preferred, and for example, steroids such as prednisolone, betamethasone, triamcinolone, triamcinolone acetonide, budesonide, and fluticasone can be exemplified. Further, examples of API other than the steroids include nonsteroidal anti-inflammatory drugs such as naproxen and diclofenac.

The molecular weight of API may be, for example, 600 or less, and is preferably 500 or less.

In an embodiment, API having a molecular weight of 600 or less and CS having a weight average molecular weight of 5,000 or more are used. In another embodiment, API having a molecular weight of 500 or less and CS having a weight average molecular weight of 5,000 or more are used.

In an embodiment, the hydrophobic compound is API or a compound in which API and a spacer forming molecule are covalently bonded to each other. The spacer forming molecule preferably forms the hydrophobic compound by being covalently bonded to API. Further, the spacer forming molecule preferably covalently bonded to CS.

The hydrophobic compound preferably has a ClogP of 1 or more. That is, in a preferred embodiment, a compound having a ClogP of 1 or more is used as the hydrophobic compound. By setting the ClogP of the hydrophobic compound to 1 or more, when the chondroitin sulfate derivative is administered to the lumen of the bladder of a subject, it is possible to facilitate effective delivery of API to a submucosal tissue of the bladder. The upper limit of the ClogP is not limited, but can be, for example, 10 or less, or 5 or less.

Here, the "log P" in the present description is a partition coefficient based on the ratio of the concentration of a compound distributed in each of a two-phase system of water and octanol. The log P is an index indicating the hydrophobicity of a compound, and as the hydrophobicity is larger, the value of the log P becomes larger. The "ClogP" is a log P value calculated by calculation using a fragment method (A. Leo, Comprehensive Medicinal Chemistry; Hansch C., Ed., Pergamon Press, Oxford, 1990, Vol. 4, p. 315; Leo A. J., Chemical Reviews, 1993, Vol. 93, pp. 1281-1306). The ClogP can be calculated using Chem Draw Professional ver.18.1.2.18 (PerkinElmer, Inc.).

As understood by a person skilled in the art, a transitional epithelium (or also referred to as mucosal epithelial layer) present closest to the lumen of the bladder is a multilayer constituted by three layers including a superficial layer (including tegmental cells), an intermediate layer (intermediate cell layer), and a basal layer (basal cell layer) from the lumen side. In the present description, the "submucosal tissue" of the bladder indicates a portion on the parenchymal side of the transitional epithelium (the parenchymal side indicates a direction farther from the lumen in a tissue section image of the bladder sectioned perpendicularly to an axis connecting the top of the bladder to the urethra), and includes a lamina propria, a submucosal layer, a muscle layer, and a serous membrane from the lumen side. The chondroitin sulfate derivative administered to the lumen of the bladder can deliver API to the lamina propria or to the parenchymal side from the lamina propria.

API is preferably released from the chondroitin sulfate derivative after being administered to the lumen of the bladder. By the release of API from the chondroitin sulfate derivative after being administered to the lumen of the bladder, API can be released in a sustained manner after permeating into a submucosal tissue of the bladder. According to this, in this embodiment, a particularly excellent effect can be exerted as a bladder disease treatment agent. From the viewpoint of obtaining such a function, the bond between API and the spacer forming molecule is preferably a covalent bond that is degradable in a living body, and is formed by a condensation reaction, and more preferably an ester bond. By degradation (preferably hydrolysis) of the covalent bond between API and CS or the spacer forming molecule after administering the chondroitin sulfate derivative to the lumen of the bladder, API is released. Note that after the bond between CS and the spacer forming molecule is degraded, the covalent bond between API and the spacer forming molecule may be degraded to release API. In an embodiment, API is released from the chondroitin sulfate derivative over a period of 168 hours (7 days) or more after being administered to the lumen of the bladder.

In an embodiment, in the chondroitin sulfate derivative, API is covalently bonded to the spacer forming molecule through an ester bond or an amide bond. In that case, the spacer forming molecule is more preferably a compound containing two or more functional groups selected from a carboxy group, a hydroxy group, and an amino group in the molecular structure.

In the chondroitin sulfate derivative, the spacer forming molecule is preferably covalently bonded to CS through an amide bond. Further, from the viewpoint of controlling the release rate of API, API is preferably covalently bonded to the spacer forming molecule through an ester bond. Therefore, the spacer forming molecule is preferably a compound containing a carboxy group and an amino group or a hydroxy group and an amino group in the molecular structure.

When the spacer forming molecule is a compound containing a carboxy group and an amino group in the molecular structure, a carboxy group of CS and an amino group of the spacer forming molecule are bonded through an amide bond, and a hydroxy group of API and a carboxy group of the spacer forming molecule are bonded through an ester bond. On the other hand, when the spacer forming molecule is a compound containing a hydroxy group and an amino group in the molecular structure, a carboxy group of CS and an amino group of the spacer forming molecule are bonded through an amide bond, and a carboxy group of API and a hydroxy group of the spacer forming molecule are bonded through an ester bond.

An example of the spacer forming molecule can be represented by the following general formula (I).

$$H_2N\text{-}(CH_2)_p\text{-}X \qquad (I)$$

In the formula, p represents an integer of 1 to 12; each $-CH_2-$ may independently have a substituent; and X represents a hydroxy group, a carboxy group, or an amino group. p is preferably an integer of 1 to 6, more preferably 2 to 6. The substituent can be a group selected from the group consisting of an electron donating group and a sterically hindering group. In an embodiment, $-N_2H$ represented by the general formula (I) and a carboxy group of CS form an amide bond.

The "electron donating group" used in the present description means a group (for example, an alkyl group, an aryl group, or the like) that donates an electron more easily than a hydrogen atom. Further, the "sterically hindering group" used in the present description means a bulky group that causes steric hindrance in a chemical reaction such as solvolysis. When a methylene group in the general formula (I) is substituted with an electron donating group or a sterically hindering group, it become difficult to degrade the covalent bond between API and the spacer forming molecule, and the release rate of API decreases. The decrease in the release rate can be appropriately measured by the below-mentioned method.

The electron donating group or the sterically hindering group are not particularly limited as long as they can be substituted for a methylene group in the general formula (I), and can be appropriately selected from generally used electron donating groups or sterically hindering groups. Specifically, a straight-chain alkyl group having 1 to 12 carbon atoms such as a methyl group, an ethyl group, a propyl group, or a butyl group; a branched-chain alkyl group having 3 to 12 carbon atoms such as an isopropyl group, an isobutyl group, a sec-butyl group, or a tert-butyl group; a cyclic alkyl group having 3 to 6 carbon atoms such as a cyclohexyl group; an aryl group having 6 to 10 carbon atoms such as a phenyl group or a tolyl group; an alkenyl group having 2 to 12 carbon atoms such as a vinyl group or an allyl group; an alkynyl group having 2 to 12 carbon atoms; an aralkyl group having 7 to 12 carbon atoms such as a benzyl group, and the like can be exemplified. Among these, a group selected from the group consisting of a methyl group, a branched-chain alkyl group having 3 to 4 carbon atoms, a benzyl group, and the like is preferred. As the spacer forming molecule, specifically, an amino acid such as β-alanine, isoleucine, alanine, valine, leucine, or phenylalanine; an aminoalkyl alcohol having 2 to 12 carbon atoms such as 2-aminoethanol or 3-aminopropanol, or the like can be used.

The spacer forming molecule exists as a group derived from the spacer forming molecule (that is a spacer) in the chondroitin sulfate derivative. Therefore, the chondroitin sulfate derivative contains at least one type of group derived from the spacer forming molecule. In an embodiment, the group derived from the spacer forming molecule is a linking group formed by removing a hydrogen atom from an amino group contained in the spacer forming molecule, and also removing a hydroxy group from a carboxy group contained in the spacer forming molecule or hydrogen from a hydroxy group contained in the spacer forming molecule. As the group derived from the spacer forming molecule, an amino acid residue represented by $-HN-(CH_2)_2-CO-$, $-HN-CH(CH(CH_3)CH_2CH_3)-CO-$, $-HN-CH(CH(CH_3)_2)$ $-CO-$, or $-HN-CHCH_2(CH(CH_3)_2)-CO-$; or an amino alcohol represented by $-HN-(CH_2)_2-O-$, $-HN-CH(CH(CH_3)CH_2CH_3)-O-$, $-HN-CH(CH(CH_3)_2)-O-$, or $-HN-CHCH_2(CH(CH_3)_2)$ $-O-$ is preferred, and $-HN-$ $(CH_2)$ $_2-CO-$, $-HN-$ $(CH_2)_2-O-$, $-HN-CH(CH(CH_3)CH_2CH_3)-CO-$, or $-HN-CH(CH(CH_3)CH_2CH_3)-O-$ is more preferred.

The release rate of API from the chondroitin sulfate derivative can be appropriately selected according to the intended purpose. The release rate of API is, for example in a 10 mM phosphate buffer solution (pH 7.4) at 36°C, preferably from 0.1 to 5 %/day, and more preferably from 0.1 to 3 %/day. Here, the release rate of API is the amount of change in the release ratio (%) per day and is defined as a slope of a graph when the time is plotted on the horizontal axis and the release ratio (%) that is the ratio of the release amount (mol) of API with respect to the total number of moles (100%) of the group derived from API contained in the chondroitin sulfate derivative is plotted on the vertical axis.

Therefore, when the amount of change in the release ratio maintains a constant value during the measurement period, the graph becomes a monotonically increasing straight line. In that case, for example, when all API contained in the chondroitin sulfate derivative is released in 10 days in a 10 mM phosphate buffer solution (pH 7.4) at 36°C, the release rate is expressed by 10 %/day.

On the other hand, when the amount of change in the release ratio does not show a constant value and decreases over time (the graph shows a gentle curve that is convex upward and is monotonically increasing), the release rate is calculated by linearly approximating the change in the release ratio during the initial start-up period (for example, 3 days).

Note that the release amount of API (the amount of released API) is measured by a method appropriately selected according to the type of API.

(2) Method for Producing Chondroitin Sulfate Derivative

A method for producing the chondroitin sulfate derivative is not particularly limited. For example, the chondroitin

sulfate derivative can be obtained by covalently bonding a functional group contained in CS to a functional group contained in a hydrophobic compound (for example, a spacer forming molecule or API). In an embodiment, the chondroitin sulfate derivative can be obtained by covalently bonding a carboxy group contained in CS to a hydroxy group or an amino group contained in a hydrophobic compound.

Hereinafter, an example of the method for producing the chondroitin sulfate derivative will be described, but the method is not limited to the following method.

The chondroitin sulfate derivative in which CS and API are covalently bonded through a spacer forming molecule can be produced by, for example, a production method including the following steps. A production method including:

(A) a step of binding a hydroxy group contained in API to a carboxy group of a spacer forming molecule through a covalent bond (ester bond) by condensation, or binding a carboxy group contained in API to a hydroxy group of a spacer forming molecule through a covalent bond (ester bond) by condensation, thereby obtaining a hydrophobic compound; and

(B) a step of binding a carboxy group contained in CS to an amino group of the spacer forming molecule through a covalent bond (amide bond) by condensation.

In the step (A), a hydrophobic compound is obtained by binding a hydroxy group contained in API to a carboxy group of a spacer forming molecule through a covalent bond by condensation, or binding a carboxy group contained in API to a hydroxy group of a spacer forming molecule through a covalent bond (ester bond) by condensation. At that time, the amino group to be reacted with CS of the spacer forming molecule may be protected by a commonly used method as needed.

In the step (B), a carboxy group contained in CS and an amino group of the spacer forming molecule are covalently bonded to each other by condensation. At that time, the carboxy group or the hydroxy group to be reacted with API of the spacer forming molecule may be protected by a commonly used method as needed.

The method for producing the chondroitin sulfate derivative needs only to include the step (A) and the step (B), and the order of the steps to be performed is not limited.

The condensation (esterification or amidation) method may be appropriately selected from commonly used methods. As the condensation method, for example, a method using a condensing agent such as a water-soluble carbodiimide (for example, 1-(3-dimethylaminopropyl)-3-ethylcarbodiimide hydrochloride), 4-(4,6-dimethoxy-1,3,5-triazin-2-yl)-4-methylmorpholinium chloride (DMT-MM), or dicyclohexylcarbodiimide, a symmetric acid anhydride method, a mixed acid anhydride method, an active ester method, and the like can be exemplified. The condensation reaction conditions are appropriately selected according to the condensation reaction to be applied.

The spacer forming molecule is preferably selected so that a desired release rate of API is obtained. In an embodiment, the spacer forming molecule has a carboxy group or a hydroxy group that forms an ester bond with API, and an amino group that forms an amide bond with CS, and a linking group that links two reactive functional groups. The linking group is preferably selected so that the degradation rate of the ester bond with API is controlled.

(3) Administration Method

The chondroitin sulfate derivative is administered to the lumen of the bladder of a subject. By administering the chondroitin sulfate derivative in this manner, API can be transmucosally delivered to a submucosal tissue of the bladder. As a specific administration method, for example, a method in which a urethral catheter is inserted into the lumen of the bladder under aseptic conditions to evacuate residual urine, and thereafter, the chondroitin sulfate derivative is injected through the same catheter, or the like can be exemplified. The bladder may be one with a damaged mucosa. The subject can be a human or a non-human animal such as a mouse, a rat, a rabbit, a dog, or a pig.

The chondroitin sulfate derivative injected into the lumen of the bladder reaches the lumen of the bladder and adheres to the mucosa while keeping the state where CS and the hydrophobic compound are covalently bonded to each other. Thereafter, the chondroitin sulfate derivative permeates into a submucosal tissue of the bladder.

In the case where the chondroitin sulfate derivative is administered using, for example, a urethral catheter, the concentration of the chondroitin sulfate derivative when the chondroitin sulfate derivative is used as the following pharmaceutical composition in a liquid state and administered needs only to be a concentration capable of passing through the catheter, and can be appropriately adjusted by a person skilled in the art.

(4) Pharmaceutical Composition

The chondroitin sulfate derivative can be used as a pharmaceutical composition. The pharmaceutical composition contains at least one type of chondroitin sulfate derivative, and may further contain another component such as a pharmaceutically acceptable excipient as needed.

(5) Bladder Disease Treatment Agent

The chondroitin sulfate derivative can be used as a bladder disease treatment agent. The bladder disease treatment agent is preferably formed into a pharmaceutical composition containing at least one type of chondroitin sulfate derivative, and the pharmaceutical composition is used for a treatment of a bladder disease.

The "treatment" used in the present description may only be some sort of treatment to be conducted for a disease, and for example, therapy, improvement, and suppression of progression (prevention of aggravation) of a disease are exemplified. Further, the "bladder disease" in the present description means a disease and other some sort of abnormalities occurring in the bladder, and for example, cystitis and the like are included.

The form of the bladder disease treatment agent is not particularly limited as long as it is the form of a drug formulation or a medicine that can be administered to the lumen of the bladder of a human, however, the form when administering is preferably a liquid, and for example, the form of a solution, a suspension, or the like is exemplified. The solution and the suspension can also be administered by dissolving a powder of the chondroitin sulfate derivative before use to prepare them.

(6) Method for Treating Bladder Disease

A method for treating a bladder disease includes a step of administering the above-mentioned delivery system to the lumen of the bladder of a subject. Specifically, the method for treating a bladder disease includes a step of administering the chondroitin sulfate derivative to be used as the bladder disease treatment agent to the lumen of the bladder of a subject. The method for treating a bladder disease may further include another step as needed. The administration of the bladder disease treatment agent can be carried out similarly according to the description of "(3) Administration Method".

According to the first embodiment of the present invention, it is possible to achieve effective delivery of a pharmaceutical active ingredient administered to the lumen of the bladder to a submucosal tissue.

(Second Embodiment)

A second embodiment of the present invention relates to a method for screening a drug candidate compound for treating a bladder disease. The method includes a step of providing a compound, a step of administering the compound to the lumen of the bladder, and a step of analyzing the presence of the compound in a submucosal tissue of the bladder, wherein the presence of the compound in the submucosal tissue of the bladder is used as an index of the drug candidate compound.

The compound (test substance) to be subjected to the method may be any compound, but is preferably a compound expected to have drug efficacy against a bladder disease.

The lumen of the bladder to which the compound is administered may be the lumen of the bladder of an animal (for example, a rat, a mouse, a guinea pig, a dog, or a monkey) in which a bladder disease is induced. The method for administering the compound to the lumen of the bladder is not particularly limited, but for example, a method in which a urethral catheter is inserted into the lumen of the bladder under aseptic conditions to evacuate residual urine, and thereafter, the compound is injected through the same catheter, or the like can be exemplified. The dose of the compound needs only be an amount sufficient for coating the entire mucosa of the lumen of the bladder, and may be appropriately set according to the type of animal.

The method for analyzing the presence of the compound in a submucosal tissue of the bladder is not particularly limited, and a known method can be used. The analytical method may be appropriately selected from imaging, mass spectrometry, an immunoassay, radioactivity measurement, and the like according to the type of compound. These methods may be used singly, or two or more methods may be used in combination.

Here, as one example, a method for analyzing the presence of the compound in a submucosal tissue of the bladder using imaging, particularly, fluorescent imaging will be described.

When fluorescent imaging is used, luminescence of the compound in a submucosal tissue is optically detected. In order to detect luminescence of the compound, it is necessary to fluorescently label the compound of the test substance in advance. The fluorescent labeling method is not particularly limited, but a method of introducing a fluorescent dye compound into the compound of the test substance, or the like may be used. For example, when the chondroitin sulfate derivative containing API is used as the compound of the test substance, a fluorescent dye such as fluorescein may be introduced into a functional group of API or CS. The test substance is preferably one having a polymeric structure such as a polymer.

The presence of the compound in a submucosal tissue can be analyzed by observing a frozen section with a fluorescence microscope after collecting the bladder of an animal and preparing the frozen section. The conditions such as the excitation wavelength and the absorption wavelength of the fluorescence microscope, or the like may be appropriately adjusted according to the type of the introduced fluorescent dye.

The presence of the compound in a submucosal tissue is preferably analyzed 6 hours or more after administering the compound to the lumen of the bladder. By performing the analysis after 6 hours or more, it becomes easy to confirm the presence of the compound that permeated into a submucosal tissue from the mucosa of the bladder.

The presence of the compound in a submucosal tissue may be evaluated by calculating an integrated value of the luminance from the fluorescent image. For example, with respect to the compound effective against a bladder disease, an integrated value of the luminance from the fluorescent image is determined in advance and defined as an evaluation criterion, and a compound having a larger integrated value of the luminance than the evaluation criterion may be determined as the drug candidate compound.

The drug candidate compound screened by the above-mentioned method can be used as a drug for treating a bladder disease (a bladder disease treatment agent).

The above-mentioned method can be used in a method for producing a drug for treating a bladder disease (a bladder disease treatment agent). That is, the method for producing a drug for treating a bladder disease (a bladder disease treatment agent) is included in the method for screening a drug candidate compound for treating a bladder disease (hereinafter referred to as "screening step").

Further, the method for producing a drug for treating a bladder disease (a bladder disease treatment agent) may include a step of synthesizing or extracting the compound that is a test substance before the screening step.

According to the second embodiment of the present invention, a drug candidate compound effective in treating a bladder disease can be appropriately screened.

(Third Embodiment)

A third embodiment of the present invention relates to a method for giving drug efficacy to a submucosal tissue of the bladder. The method includes a step of administering a chondroitin sulfate derivative in which chondroitin sulfate and a hydrophobic compound are bound to each other to the lumen of the bladder of a subject.

As the chondroitin sulfate derivative, the same chondroitin sulfate derivative as described in the first embodiment of the present invention can be used. Further, the method for administering the chondroitin sulfate derivative can also be carried out in the same manner as the administration method described in the first embodiment of the present invention.

The third embodiment of the present invention can be used in the method for treating a bladder disease. That is, the method for treating a bladder disease includes the method for giving drug efficacy to a submucosal tissue of the bladder. Specifically, the method for treating a bladder disease includes a step of administering the chondroitin sulfate derivative to be used as a bladder disease treatment agent to the lumen of the bladder of a subject. The method for treating a bladder disease may further include another step as needed.

According to the third embodiment of the present invention, the pharmaceutical active ingredient administered to the lumen of the bladder can be made to effectively exhibit drug efficacy in a submucosal tissue of the bladder.

Examples

Hereinafter, Examples and Test Examples of the present invention will be more specifically described in detail. However, the technical scope of the present invention is not limited thereto.

(Example 1)

(1-1) Preparation of 21-(Boc-β-alanyl)-triamcinolone acetonide

2.63 g of Boc-β-alanine (manufactured by Watanabe Chemical Industries, Ltd.) was dissolved in 40 mL of dichloromethane and 10 mL of dimethylformamide, and 5.01 g of triamcinolone acetonide (manufactured by Wako Pure Chemical Industries, Ltd., hereinafter also abbreviated as "TA") was added thereto. Thereafter, 426 mg of 4-dimethylaminopyridine and 3.32 g of a water-soluble carbodiimide were added thereto under ice cooling, followed by stirring overnight at room temperature. After confirming the disappearance of the raw materials by thin-layer chromatography, a saturated aqueous ammonium chloride solution was added to stop the reaction. Two-liquid extraction was performed using toluene and water, and the collected organic layer was washed sequentially with a saturated aqueous ammonium chloride solution, a saturated aqueous sodium bicarbonate solution, and a saturated aqueous sodium chloride solution. Thereafter, the organic layer was dried over magnesium sulfate and filtered, and then, the solvent was distilled off under reduced pressure, whereby 6. 66 g of target 21-(Boc-β-alanyl)-triamcinolone acetonide was obtained (yield: 96%).

(1-2) Preparation of 21-β-alanyl-triamcinolone acetonide hydrochloride

6.66 g of 21- (Boc-β-alanyl) -triamcinolone acetonide was dissolved in 90 mL of tetrahydrofuran, and 90 mL of 4 M hydrochloric acid/ethyl acetate was added thereto under ice cooling, followed by stirring at room temperature for 3 hours. After confirming the disappearance of the raw material by thin-layer chromatography, the solvent was distilled off under reduced pressure. The concentrate was washed with diethyl ether, and the solvent was distilled off under reduced pressure, whereby 5.78 g of target 21-β-alanyl-triamcinolone acetonide hydrochloride was obtained (yield: 97%).

Note that 21- (β-alanyl)-triamcinolone acetonide has the following structure, and as a result of calculation of ClogP using Chem Draw Professional ver.18.1.2.18 (PerkinElmer, Inc.), it was 2.17.

[Chem. 1]

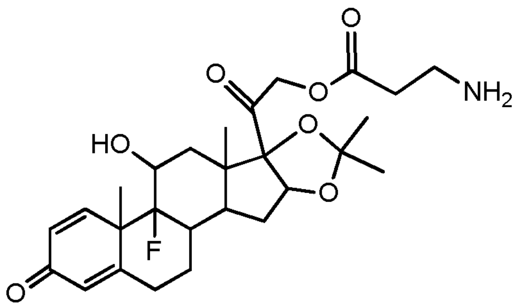

(1-3) Preparation of 21-β-alanyl-triamcinolone acetonide-introduced CS (hereinafter abbreviated as "CS-TA-conjugate")

524 mg of CS (sodium salt, manufactured by Seikagaku Corporation, weight average molecular weight measured by light scattering method: about 20,000) was dissolved in 10 mL of distilled water, and 10 mL of acetone was added thereto, followed by stirring. 458 mg of 21-β-alanyl-triamcinolone acetonide hydrochloride and 839 mg of 4-(4,6-dimethoxy-1,3,5-triazin-2-yl)-4-methylmorpholinium chloride hydrate (hereinafter DMT-MM) (manufactured by Tokuyama Corporation) were added thereto while rinsing out the container with 50% (v/v) acetone/distilled water (3 mL in total), followed by stirring overnight. Thereafter, the reaction solution in which 0.1 g of sodium chloride and 50% (v/v) acetone/distilled water that was used for rinsing out the container were added, and sodium chloride was dissolved was sequentially added to 300 mL of 90% (v/v) ethanol/distilled water to form a precipitate, and then, the supernatant was discarded. Thereafter, the precipitate was washed sequentially with 90% (v/v) ethanol/distilled water and 95% (v/v) ethanol/distilled water. The obtained precipitate was dried overnight under reduced pressure, whereby 819 mg of a target CS-TA-conjugate was obtained.

(1-4) Preparation of fluorescently labeled 21-β-alanyl-triamcinolone acetonide-introduced CS (hereinafter abbreviated as "fluorescently labeled CS-TA-conjugate")

500 mg of the CS-TA-conjugate was dissolved in 5.5 mL of distilled water, and 5.5 mL of acetone was added thereto, followed by stirring. 5 mg of fluoresceinyl glycine amide (manufactured by Setareh Biotech, LLC.) and 24 mg of DMT-MM were added thereto while rinsing out the container with 50% (v/v) acetone/distilled water (10 mL in total), followed by stirring overnight. Thereafter, to a solution obtained by dissolving 5.0 g of sodium chloride in 37.5 mL of distilled water and adding 125 mL of 2-propanol thereto, the reaction solution was added while rinsing out the container with 9 mL of 50% (v/v) acetone/distilled water. Thereafter, 25 mL of 2-propanol was added thereto to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 471 mg of a target fluorescently labeled CS-TA-conjugate was obtained.

(Example 2)

(2-1) Preparation of (Boc-β-alanyl)-hydroxyzine

2.00 g of hydroxyzine dihydrochloride (manufactured by FUJIFILM Wako Pure Chemical Corporation, hereinafter also abbreviated as "HYD"), 1.09 g of 4-dimethylaminopyridine (manufactured by FUJIFILM Wako Pure Chemical Corporation) , and 0.846 g of Boc-β-alanine (manufactured by Watanabe Chemical Industries, Ltd.) were added to 20

mL of dichloromethane and dissolved therein. Thereafter, 1.71 g of a water-soluble carbodiimide (manufactured by Tokyo Chemical Industry Co., Ltd.) was added thereto, followed by stirring overnight at room temperature. After confirming the disappearance of the raw materials by thin-layer chromatography, stirring was stopped, and the reaction solution was washed sequentially with a 5% (w/v) aqueous sodium bicarbonate solution and a 20% (w/v) aqueous sodium chloride solution. Thereafter, the reaction solution was dehydrated with sodium sulfate and filtered, and then, the solvent was distilled off under reduced pressure. The concentrate was purified with a silica gel column (chloroform:methanol), whereby 1.99 g of target (Boc-β-alanyl)-hydroxyzine was obtained (yield: 82%).

(2-2) Preparation of β-alanyl-hydroxyzine trihydrochloride

1.93 g of (Boc-β-alanyl)-hydroxyzine was dissolved in 71 mL of dichloromethane, and 19.4 mL of 4 mol/L-HCl/AcOEt (manufactured by Watanabe Chemical Industries, Ltd.) was added thereto under ice cooling, followed by stirring under ice cooling for 110 minutes. After confirming the disappearance of the raw material by thin-layer chromatography, the solvent was distilled off under reduced pressure. After the obtained oily substance was dissolved in dichloromethane, methyl tert-butyl ether was added thereto to precipitate a crystal. The crystal was dried overnight at 40°C under reduced pressure, whereby 1.45 g of target β-alanyl-hydroxyzine trihydrochloride was obtained (yield: 74%).

Note that β-alanyl-hydroxyzine has the following structure, and as a result of calculation of ClogP using Chem Draw Professional ver.18.1.2.18 (PerkinElmer, Inc.), it was 4.31.

[Chem. 2]

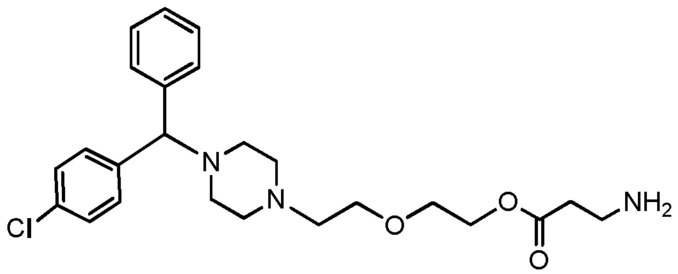

(2-3) Preparation of β-alanyl-hydroxyzine-introduced CS (hereinafter abbreviated as "CS-HYD-conjugate")

1.04 g of CS (sodium salt, manufactured by Seikagaku Corporation, weight average molecular weight measured by light scattering method: about 20,000) was dissolved in 10 mL of distilled water, and 10 mL of acetone was added thereto, followed by stirring. 111 mg of β-alanyl-hydroxyzine trihydrochloride and 167 mg DMT-MM (manufactured by Tokuyama Corporation) were added thereto while rinsing out the container with 50% (v/v) acetone/distilled water (20 mL in total), followed by stirring overnight. Thereafter, 2.00 g of sodium chloride was added thereto and dissolved therein, and then, the reaction solution was added to 80 mL of 95% (v/v) ethanol/distilled water while rinsing out the container with 15 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 903 mg of a target CS-HYD-conjugate was obtained.

(2-4) Preparation of fluorescently labeled β-alanyl-hydroxyzine-introduced CS (hereinafter abbreviated as "fluorescently labeled CS-HYD-conjugate")

500 mg of the CS-HYD-conjugate was dissolved in 5 mL of distilled water, and 5 mL of acetone and 5 mL of 50% (v/v) acetone/distilled water were added thereto, followed by stirring. 5 mg of fluoresceinyl glycine amide (manufactured by Setareh Biotech, LLC.) and 24 mg of DMT-MM were added thereto while rinsing out the container with 50% (v/v) acetone/distilled water (5 mL in total), followed by stirring overnight. Thereafter, 0.75 g of sodium chloride was added thereto and dissolved therein. The reaction solution was added to 40 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 448 mg of a target fluorescently labeled CS-HYD-conjugate was obtained.

(Example 3)

(3-1) Preparation of (Boc-2-aminoethyl)-naproxen

1.06 g of N-Boc-ethanolamine (manufactured by Tokyo Chemical Industry Co., Ltd.), 1.50 g of (S)-naproxen (manufactured by FUJIFILM Wako Pure Chemical Corporation, hereinafter also abbreviated as "NAP"), and 1.59 g of 4-dimethylaminopyridine (manufactured by FUJIFILM Wako Pure Chemical Corporation) were added to 28 mL of dichloromethane and dissolved therein. Thereafter, 2.50 g of a water-soluble carbodiimide (manufactured by Tokyo Chemical Industry Co., Ltd.) was added thereto, followed by stirring overnight at room temperature. After confirming the disappearance of the raw materials by thin-layer chromatography, stirring was stopped, and the reaction solution was washed sequentially with a 10% (w/v) aqueous potassium hydrogen sulfate solution, a 5% (w/v) aqueous sodium bicarbonate solution, and a 20% (w/v) aqueous sodium chloride solution. Thereafter, the reaction solution was dehydrated with magnesium sulfate and filtered, and then, the solvent was distilled off under reduced pressure, whereby 2.32 g of target (Boc-2-aminoethyl)-naproxen was obtained (yield: 95%).

(3-2) Preparation of 2-aminoethyl-naproxen hydrochloride

2.15 g of (Boc-2-aminoethyl)-naproxen was dissolved in 115 mL of dichloromethane, and 31.6 mL of 4 mol/L-HCl/AcOEt (manufactured by Watanabe Chemical Industries, Ltd.) was added thereto under ice cooling, followed by stirring at room temperature for 1.5hours. After confirming the disappearance of the raw material by thin-layer chromatography, a crystal precipitated in the reaction solution was obtained by filtration. The crystal was dried overnight at 40°C under reduced pressure, whereby 1.42 g of target 2-aminoethyl-naproxen hydrochloride was obtained (yield: 80%) .

Note that 2-aminoethyl-naproxen has the following structure, and as a result of calculation of ClogP using Chem Draw Professional ver.18.1.2.18 (PerkinElmer, Inc.), it was 2.62.

[Chem. 3]

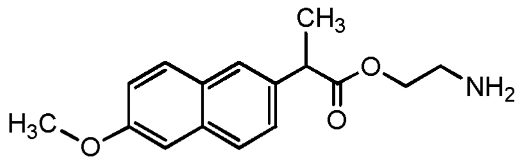

(3-3) Preparation of 2-aminoethyl-naproxen-introduced CS (hereinafter abbreviated as "CS-NAP-conjugate")

1.04 g of CS (sodium salt, manufactured by Seikagaku Corporation, weight average molecular weight measured by light scattering method: about 20,000) was dissolved in 10 mL of distilled water, and 10 mL of acetone was added thereto, followed by stirring. 60 mg of 2-aminoethyl-naproxen hydrochloride and 167 mg DMT-MM (manufactured by Tokuyama Corporation) were added thereto while rinsing out the container with 50% (v/v) acetone/distilled water (20 mL in total), followed by stirring overnight. Thereafter, 2.00 g of sodium chloride was added thereto and dissolved therein, and then, the reaction solution was added to 80 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 1.03 g of a target CS-NAP-conjugate was obtained.

(3-4) Preparation of fluorescently labeled 2-aminoethyl-naproxen-introduced CS (hereinafter abbreviated as "fluorescently labeled CS-NAP-conjugate")

500 mg of the CS-NAP-conjugate was dissolved in 5 mL of distilled water, and 5 mL of acetone and 5 mL of 50% (v/v) acetone/distilled water were added thereto, followed by stirring. 5 mg of fluoresceinyl glycine amide (manufactured by Setareh Biotech, LLC.) and 24 mg of DMT-MM were added thereto while rinsing out the container with 50% (v/v) acetone/distilled water (5 mL in total), followed by stirring overnight. Thereafter, 0.75 g of sodium chloride was added thereto and dissolved therein, and then, the reaction solution was added to 40 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 568 mg

of a target fluorescently labeled CS-NAP-conjugate was obtained.

(Example 4)

(4-1) Preparation of (Boc-2-aminoethyl)-dehydrocholate ester

2.00 g of dehydrocholic acid (manufactured by Tokyo Chemical Industry Co., Ltd., hereinafter also abbreviated as "DEH"), 0.80 g of N-Boc-ethanolamine (manufactured by Tokyo Chemical Industry Co., Ltd.), and 1.22 g of 4-dimethylaminopyridine (manufactured by FUJIFILM Wako Pure Chemical Corporation) were added to 21 mL of dichloromethane and dissolved therein. Thereafter, 1.90 g of a water-soluble carbodiimide (manufactured by Tokyo Chemical Industry Co., Ltd.) was added thereto, followed by stirring overnight at room temperature. Stirring was stopped, and the reaction solution was washed sequentially with a 10% (w/v) aqueous potassium hydrogen sulfate solution, a 5% (w/v) aqueous sodium bicarbonate solution, and a 20% (w/v) aqueous sodium chloride solution. Thereafter, the reaction solution was dehydrated with magnesium sulfate and filtered, and then, the solvent was distilled off under reduced pressure, whereby 2.20 g of target (Boc-2-aminoethyl)-dehydrocholate ester was obtained (yield: 81%) .

(4-2) Preparation of 2-aminoethyl-dehydrocholate ester hydrochloride

2.16 g of (Boc-2-aminoethyl)-dehydrocholate ester was dissolved in 79 mL of dichloromethane, and 21.7 mL of 4 mol/L-HCl/AcOEt (manufactured by Watanabe Chemical Industries, Ltd.) was added thereto under ice cooling, followed by stirring at room temperature for 90 minutes, and then, a crystal precipitated in the reaction solution was obtained by filtration. The crystal was dried overnight at 40°C under reduced pressure, whereby 1.25 g of target 2-aminoethyl-dehydrocholate ester hydrochloride was obtained (yield: 66%).

Note that 2-aminoethyl-dehydrocholate has the following structure, and as a result of calculation of ClogP using Chem Draw Professional ver.18.1.2.18 (PerkinElmer, Inc.), it was 2.03.

[Chem. 4]

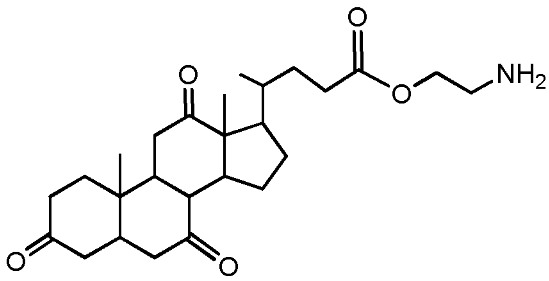

(4-3) Preparation of 2-aminoethyl-dehydrocholate-introduced CS (hereinafter abbreviated as "CS-DEH-conjugate")

1.04 g of CS (sodium salt, manufactured by Seikagaku Corporation, weight average molecular weight measured by light scattering method: about 20,000) was dissolved in 10 mL of distilled water, and 10 mL of acetone was added thereto, followed by stirring. 96 mg of 2-aminoethyl-dehydrocholate ester hydrochloride and 167 mg of DMT-MM (manufactured by Tokuyama Corporation) were added thereto while rinsing out the container with 50% (v/v) acetone/distilled water (20 mL in total), followed by stirring overnight. Thereafter, 2.00 g of sodium chloride was added thereto and dissolved therein, and then, the reaction solution was added to 80 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 1.05 g of a target CS-DEH-conjugate was obtained.

(4-4) Preparation of fluorescently labeled 2-aminoethyl-dehydrocholate-introduced CS (hereinafter abbreviated as "fluorescently labeled CS-DEH-conjugate")

500 mg of the CS-DEH-conjugate was dissolved in 5 mL of distilled water, and 5 mL of acetone and 5 mL of 50% (v/v) acetone/distilled water were added thereto, followed by stirring. 5 mg of fluoresceinyl glycine amide (manufactured by Setareh Biotech, LLC.) and 24 mg of DMT-MM were added thereto while rinsing out the container with 50% (v/v) acetone/distilled water (5 mL in total), followed by stirring overnight. Thereafter, 0.75 g of sodium chloride was

added thereto and dissolved therein, and then, the reaction solution was added to 40 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 571 mg of a target fluorescently labeled CS-DEH-conjugate was obtained.

(Example 5)

(5-1) Preparation of Boc-2-aminoethyl bromide

2.155 g of 2-bromoethylamine hydrobromide was dissolved in 20 mL of dichloromethane, and 1.463 mL of triethylamine was added thereto under ice cooling, and 5 mL of a dichloromethane solution of 2.299 g of $Boc_2O$ was further added thereto, followed by stirring. After the resulting mixture was stirred at room temperature for 90 minutes, ethyl acetate was added thereto, and the resulting mixture was sequentially subjected to two-liquid washing with a 5% (w/v) aqueous citric acid solution, water, and a saturated aqueous sodium chloride solution. The reaction solution was dehydrated with sodium sulfate, and then, the solvent was distilled off under reduced pressure, whereby 2.287 g of target Boc-2-aminoethyl bromide was obtained (yield: 97%).

(5-2) Preparation of 2-aminoethyl-diclofenac hydrochloride

5 mL of a DMF solution of 2.287 g of Boc-2-aminoethyl bromide was ice-cooled, and 6 mL of a DMF solution of 3.255 g of diclofenac sodium was added thereto, followed by stirring overnight at room temperature. The resulting mixture was stirred at 60°C for 11 hours, and then, stirred overnight at room temperature. Ethyl acetate was added thereto, and the reaction solution was sequentially subjected to two-liquid washing with a 5% (w/v) aqueous sodium bicarbonate solution, water, and a saturated aqueous sodium chloride solution. The reaction solution was dehydrated with sodium sulfate, and thereafter, ethyl acetate was distilled off under reduced pressure. The residue was purified by silica gel column chromatography (toluene: ethyl acetate =20:1 (v/v), 0.5% (v/v) triethylamine), whereby 2.675 g of Boc-2-aminoethyl-diclofenac was obtained.

Subsequently, 2.108 g of Boc-2-aminoethyl-diclofenac was dissolved in 5 mL of dichloromethane, and 20 mL of 4 M hydrochloric acid/ethyl acetate was added thereto under ice cooling, followed by stirring for 2.5 hours. Diethyl ether and hexane were added thereto to deposit a precipitate, and the precipitate was dried under reduced pressure, whereby 1.775 g of target 2-aminoethyl-diclofenac hydrochloride was obtained (yield: 98%).

Note that 2-aminoethyl-diclofenac has the following structure, and as a result of calculation of ClogP using Chem Draw Professional ver.18.1.2.18 (PerkinElmer, Inc.), it was 4.38.

[Chem. 5]

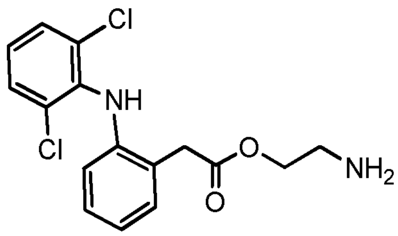

(5-3) Preparation of 2-aminoethyl-diclofenac-introduced CS (hereinafter abbreviated as "CS-DIC-conjugate")

1.04 g of CS (sodium salt, manufactured by Seikagaku Corporation, weight average molecular weight measured by light scattering method: about 20,000) was dissolved in 10 mL of distilled water, and 10 mL of acetone was added thereto, followed by stirring. 73 mg of 2-aminoethyl-diclofenac hydrochloride and 167 mg of DMT-MM (manufactured by Tokuyama Corporation) were added thereto while rinsing out the container with 50% (v/v) acetone/distilled water (20 mL in total), followed by stirring overnight. Thereafter, 2.00 g of sodium chloride was added thereto and dissolved therein, and then, the reaction solution was added to 80 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 1.08 g of a target CS-DIC-conjugate was obtained.

(5-4) Preparation of fluorescently labeled 2-aminoethyl-diclofenac-introduced CS (hereinafter abbreviated as "fluorescently labeled CS-DIC-conjugate")

500 mg of the CS-DIC-conjugate was dissolved in 5 mL of distilled water, and 5 mL of acetone and 5 mL of 50% (v/v) acetone/distilled water were added thereto, followed by stirring. 5 mg of fluoresceinyl glycine amide (manufactured by Setareh Biotech, LLC.) and 24 mg of DMT-MM were added thereto while rinsing out the container with 50% acetone/distilled water (5 mL in total), followed by stirring overnight. Thereafter, 0.75 g of sodium chloride was dissolved therein, and then, the reaction solution was added to 40 mL of 95% (v/v) ethanol/distilled water while rinsing out the container with 5 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 572 mg of a target fluorescently labeled CS-DIC-conjugate was obtained.

(Example 6)

(6-1) Preparation of (Boc-2-aminoethyl)-probenecid

1.51 g of probenecid (manufactured by Tokyo Chemical Industry Co., Ltd., hereinafter also abbreviated as "PRO"), 0.85 g of N-Boc-ethanolamine (manufactured by Tokyo Chemical Industry Co., Ltd.), and 1.29 g of 4-dimethylaminopyridine (manufactured by FUJIFILM Wako Pure Chemical Corporation) were added to 23 mL of dichloromethane and dissolved therein. Thereafter, 2.02 g of a water-soluble carbodiimide (manufactured by Tokyo Chemical Industry Co., Ltd.) was added thereto, followed by stirring overnight at room temperature. After confirming the disappearance of the raw materials by thin-layer chromatography, stirring was stopped, and the reaction solution was washed sequentially with a 1% (w/v) aqueous potassium hydrogen sulfate solution, a 5% (w/v) aqueous sodium bicarbonate solution, and a 20% (w/v) aqueous sodium chloride solution. Thereafter, the reaction solution was dehydrated with magnesium sulfate and filtered, and then, the solvent was distilled off under reduced pressure, whereby 1.96 g of target (Boc-2-aminoethyl)-probenecid was obtained (yield: 86%).

(6-2) Preparation of 2-aminoethyl-probenecid hydrochloride

1.89 g of (Boc-2-aminoethyl)-probenecid was dissolved in 88 mL of dichloromethane, and 24.2 mL of 4 mol/L-HCl/AcOEt (manufactured by Watanabe Chemical Industries, Ltd.) was added thereto under ice cooling, followed by stirring at room temperature for 80 minutes. After confirming the disappearance of the raw material by thin-layer chromatography, the solvent was distilled off under reduced pressure. The concentrate was washed with methyl tert-butyl ether, and thereafter, the solvent was distilled off under reduced pressure. The obtained crystal was dissolved in methanol, and thereafter, methyl tert-butyl ether was added thereto to precipitate a crystal. The crystal was dried overnight at 40°C under reduced pressure, whereby 1.36 g of target 2-aminoethyl-probenecid hydrochloride was obtained (yield: 85%) .

Note that 2-aminoethyl-probenecid has the following structure, and as a result of calculation of ClogP using Chem Draw Professional ver.18.1.2.18 (PerkinElmer, Inc.), it was 2.61.

[Chem. 6]

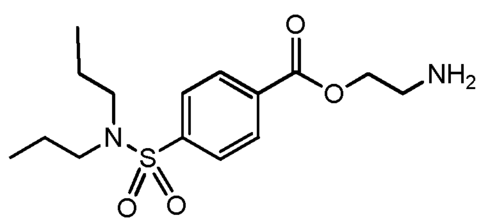

(6-3) Preparation of 2-aminoethyl-probenecid-introduced CS (hereinafter abbreviated as "CS-PRO-conjugate")

1.04 g of CS (sodium salt, manufactured by Seikagaku Corporation, weight average molecular weight measured by light scattering method: about 20,000) was dissolved in 10 mL of distilled water, and 10 mL of acetone was added thereto, followed by stirring. 71 mg of 2-aminoethyl-probenecid hydrochloride and 167 mg of DMT-MM (manufactured by Tokuyama Corporation) were added thereto while rinsing out the container with 50% (v/v) acetone/distilled water (20 mL in total), followed by stirring overnight. Thereafter, 2.00 g of sodium chloride was added thereto and dissolved therein,

and then, the reaction solution was added to 80 mL of 95% (v/v) ethanol/distilled water while rinsing out the container with 10 mL of 50% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 916 mg of a target CS-PRO-conjugate was obtained.

(6-4) Preparation of fluorescently labeled 2-aminoethyl-probenecid-introduced CS (hereinafter abbreviated as "fluorescently labeled CS-PRO-conjugate")

500 mg of the CS-PRO-conjugate was dissolved in 7.5 mL of distilled water, and 7.5 mL of acetone was added thereto, followed by stirring. 5 mg of fluoresceinyl glycine amide (manufactured by Setareh Biotech, LLC.) and 24 mg of DMT-MM were added thereto while rinsing out the container with 50% (v/v) acetone/distilled water (5 mL in total), followed by stirring overnight. Thereafter, 0.75 g of sodium chloride was added thereto and dissolved therein, and then, the reaction solution was added to 40 mL of 95% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 462 mg of a target fluorescently labeled CS-PRO-conjugate was obtained.

(Comparative Example 1)

(1-1) Preparation of fluorescently labeled CS

500 mg of CS (sodium salt, manufactured by Seikagaku Corporation, weight average molecular weight measured by light scattering method: about 20,000) was dissolved in 7.5 mL of distilled water, and 7.5 mL of acetone was added thereto, followed by stirring. 5 mg of fluoresceinyl glycine amide (manufactured by Setareh Biotech, LLC.) and 24 mg of DMT-MM were added thereto while rinsing out the container with 50% (v/v) acetone/distilled water (1.5 mL in total), followed by stirring overnight. 175 mg of sodium chloride was added thereto while rinsing out the container with 2 mL of 50% (v/v) acetone/distilled water, and thereafter, 53 mL of ethanol was added thereto to form a precipitate, and the supernatant was discarded. The precipitate was redissolved in 20 mL of distilled water, and thereafter, 100 mg of sodium chloride and 100 mL of 95% (v/v) ethanol/distilled water were added thereto to form a precipitate again, and the supernatant was discarded. Thereafter, the precipitate was washed sequentially with 95% (v/v) ethanol/distilled water and ethanol. The obtained precipitate was dried overnight at 40°C under reduced pressure, whereby 380 mg of target fluorescently labeled CS was obtained.

(Comparative Example 2)

(2-1) Preparation of 21-β-alanyl-triamcinolone acetonide-introduced HA (hereinafter abbreviated as "HA-TA-conjugate")

2.17 g of HA (sodium hyaluronate, manufactured by Seikagaku Corporation) was dissolved in 200 mL of distilled water, and 200 mL of ethanol was added thereto, followed by stirring. 272 mg of 21-β-alanyl-triamcinolone acetonide and 431 mg of DMT-MM (manufactured by Tokuyama Corporation) were added thereto while rinsing out the container with 50% (v/v) ethanol/distilled water (20 mL in total), followed by stirring overnight. Thereafter, to half the amount of the reaction solution, 11 mL of distilled water in which 0.75 g of sodium bicarbonate was dissolved was added. After the resulting mixture was left to stand for 4 hours, 0.2 mL of acetic acid was added thereto. In the reaction solution, 5.0 g of sodium chloride was dissolved, and thereafter, the resulting mixture was added to 483 mL of 90% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 929 mg of a target HA-TA-conjugate was obtained.

(2-2) Preparation of fluorescently labeled 21-β-alanyl-triamcinolone acetonide-introduced HA (hereinafter abbreviated as "fluorescently labeled HA-TA-conjugate")

500 mg of the HA-TA-conjugate was dissolved in 50 mL of distilled water, and 50 mL of ethanol was added thereto, followed by stirring. 5 mg of fluoresceinyl glycine amide (manufactured by Setareh Biotech, LLC.) and 24 mg of DMT-MM were added thereto while rinsing out the container with 50% (v/v) ethanol/distilled water (3 mL in total), followed by stirring overnight. Thereafter, to the reaction solution, 5 mL of distilled water in which 375 mg of sodium bicarbonate was dissolved was added. After the resulting mixture was left to stand for 4 hours, 0.10 mL of acetic acid was added thereto while rinsing out the container with 1 mL of distilled water. In the reaction solution, 2. 5 g of sodium

chloride was dissolved, and thereafter, the resulting mixture was added to 242 mL of 90% (v/v) ethanol/distilled water to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 567 mg of a target fluorescently labeled HA-TA-conjugate was obtained.

(Comparative Example 3)

(3-1) Preparation of 3-aminopropyl cinnamate-introduced HA (hereinafter abbreviated as "HA-CIN-conjugate")

172 mg/5 mL of N-hydroxysuccinimide (HOSu, Watanabe Chemical Industries, Ltd.) in an aqueous solution, 143 mg/5 mL of 1-ethyl-3-(3-dimethylaminopropyl)carbodiimide hydrochloride (EDCI·HCl) (Watanabe Chemical Industries, Ltd.) in an aqueous solution, and 181 mg/5 mL of 3-aminopropyl cinnamate hydrochloride (Tokyo Chemical Industry Co., Ltd.) in an aqueous solution were added to a solution of sodium hyaluronate (1.06 g, Seikagaku Corporation) in water (115 mL)/dioxane (144 mL). The resulting mixture was stirred for 3 hours, and 750 mg/10 mL of sodium bicarbonate (Japanese Pharmacopoeia) in an aqueous solution was added thereto. The mixture was further stirred for 2 hours and 30 minutes, and then, the reaction was stopped by acetic acid (214 mg) and sodium chloride (1.0 g). Ethanol (300 mL) was added thereto, and the formed precipitate was separated by filtration, and washed twice sequentially with 80% (v/v) ethanol and 95% (v/v) ethanol. The resulting solid was dried overnight at 40°C under vacuum, whereby 1.06 g of an HA-CIN-conjugate was obtained.

Note that 3-aminopropyl cinnamate has the following structure, and as a result of calculation of ClogP using Chem Draw Professional ver.18.1.2.18 (PerkinElmer, Inc.), it was 2.14.

[Chem. 7]

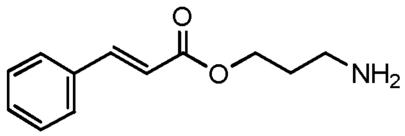

(3-2) Preparation of fluorescently labeled 3-aminopropyl cinnamate-introduced HA (hereinafter abbreviated as "fluorescently labeled HA-CIN-conjugate")

500 mg of the HA-CIN-conjugate was dissolved in 50 mL of distilled water, and 50 mL of ethanol was added thereto, followed by stirring. 5 mg of fluoresceinyl glycine amide (manufactured by Setareh Biotech, LLC.) and 24 mg of DMT-MM were dissolved in 50% (v/v) acetone/distilled water (14 mL in total) and added thereto, followed by stirring overnight. Thereafter, 375 mg of sodium bicarbonate was dissolved in 5 mL of distilled water and added thereto. 0.10 mL of acetic acid was dissolved in 3 mL of distilled water and added thereto. 2.5 g of sodium chloride was added thereto and dissolved therein, and thereafter, 242 mL of 90% (v/v) ethanol/distilled water was added thereto to form a precipitate, and the supernatant was discarded. Thereafter, the precipitate was washed sequentially with 90% (v/v) ethanol/distilled water and 95% (v/v) ethanol/distilled water. The obtained precipitate was dried overnight at 40°C under reduced pressure, whereby 462 mg of a target fluorescently labeled HA-CIN-conjugate was obtained.

<Release Test>

In Examples pertaining to a release test, a release ratio of a pharmaceutical active ingredient (API) from a chondroitin sulfate derivative and a hyaluronic acid derivative, each of which was not fluorescently labeled was evaluated as follows.

(1) Preparation of Phosphate Buffer Solution

780 mg (5.0 mmol) of sodium dihydrogen phosphate dihydrate was dissolved in 500 mL of purified water, and the resulting solution was named A solution. Separately, 1.79 g (5.0 mmol) of disodium hydrogen phosphate dodecahydrate was dissolved in 500 mL of purified water, and the resulting solution was named B solution. The A solution and the B solution were mixed at a ratio of 3:7, whereby a phosphate buffer solution at pH 7.4 was prepared.

(2) Evaluation Method

Each of the chondroitin sulfate derivatives of Examples 1 to 6 was dissolved in the phosphate buffer solution

at pH 7.4 so as to give a concentration of 5 mg/10 mL, and the resulting solution was heated for 1 week in a thermostat bath at 36°C, and the release ratio of API during the period was evaluated every 24 hours by high-performance liquid chromatography (HPLC).

The release ratio was calculated based on the ratio of peak areas of HPLC corresponding to the amount of API in the derivative and the amount of released API using a size-exclusion chromatography column (TSKgel α, manufactured by Tosoh Corporation).

Release ratio (%) = peak area of released API / (peak area of API in each derivative + peak area of released API) × 100

The detailed conditions of liquid chromatography are as follows.

Analysis time: 40 min
Flow rate: 0.5 mL/min
Gradient: isocratic
Eluent: acetonitrile (for HPLC) :physiological saline = 1:2 (v/v)
Detector: UV detector (210 nm, 230 nm, 240 nm, or 280 nm)
Temperature: 36°C

Also with respect to the hyaluronic acid derivatives of Comparative Examples 2 to 3, evaluation was performed in the same manner as for Examples.

(3) Evaluation Results

The evaluation results of the release test are shown in Table 1.

[Table 1]

| | | Example | | | | | | Comparative Example | |
|---|---|---|---|---|---|---|---|---|---|
| | | 1 | 2 | 3 | 4 | 5 | 6 | 2 | 3 |
| Compound | | CS-TA-conjugate | CS-HYD-conjugate | CS-NAP-conjugate | CS-DEH-conjugate | CS-DIC-conjugate | CS-PRO-conjugate | HA-TA-conjugate | HA-CIN-conjugate |
| elapsed time (days) | 0 | 0.10% | 0.00% | 0.08% | 0.45% | 0.00% | 0.31% | 23.23% | 0.00% |
| | 1 | 1.22% | 0.65% | 0.27% | 0.53% | 0.95% | 0.83% | 32.86% | 0.00% |
| | 2 | 1.91% | 1.48% | 0.39% | 0.52% | 2.06% | 1.43% | 40.59% | 0.00% |
| | 3 | 2.93% | 2.38% | 0.52% | 0.66% | 3.13% | 2.13% | 47.40% | 0.00% |
| | 4 | 3.92% | 3.35% | 0.68% | 0.65% | 4.11% | 2.91% | 53.06% | 0.00% |
| | 5 | 4.52% | 4.30% | 0.82% | 0.78% | 5.40% | 3.63% | 57.00% | 0.00% |
| | 6 | 5.73% | 5.24% | 0.97% | 1.29% | 6.55% | 4.56% | 61.61% | 0.00% |
| | 7 | 6.17% | 6.05% | 1.06% | 1.42% | 7.62% | 5.29% | 63.76% | 0.00% |
| Release rate | | 0.48 %/day | 0.89 %/day | 0.14 %/day | 0.14 %/day | 1.10 %/day | 0.72 %/day | 5.74 %/day | 0.00 %/day |

As shown in Table 1, the chondroitin sulfate derivatives of Examples 1 to 6 all showed a mild release rate. On the other hand, in the hyaluronic acid derivative of Comparative Example 2, a substantial release of the active ingredient was already confirmed at the start of the test. In addition, in the hyaluronic acid derivative of Comparative Example 3, release of cinnamic acid was not observed.

<Evaluation Using Rat 1>

(1) Preparation of Test Substance

Test substances were prepared by dissolving the fluorescently labeled compounds of Examples 1 to 6 and Comparative Examples 1 to 3 in phosphate-buffered saline containing 2.3% (v/v) glycerin. The concentration of each of the fluorescently labeled compounds in the test substances was set to 1% (w/v).

(2) Pretreatment of Test Animal

The lower abdomen of a rat (SD strain (SPF), female, at 7 to 9 weeks of age) was pressed under isoflurane inhalation anesthesia (concentration: 2.0 to 4.0%, flow rate: 2 to 3 L/min) to evacuate residual urine from the bladder of the rat. A catheter (PE50) was transurethrally inserted into the lumen of the bladder, and the lumen of the bladder was washed with 0.2 mL of physiological saline. Thereafter, 0.2 mL of 0.4 mol/L hydrochloric acid was injected into the lumen of the bladder and allowed to stay there for 3 minutes and 15 seconds. After allowing hydrochloric acid to stay there, the lumen of the bladder was washed four times with 0.4 mL of physiological saline.

(3) Evaluation Method

On day 1 after the pretreatment, 0.2 mL of each test substance was transurethrally administered to the lumen of the bladder using a catheter (PE50) under isoflurane inhalation anesthesia and allowed to stay there for 30 minutes.

On day 1 after the administration of the test substance, the bladder of the rat was collected, and a frozen section was prepared perpendicularly to an axis connecting the top of the bladder to the urethra. With respect to the frozen section, a bright field image (visible light) and a fluorescent image (excitation wavelength: 470 nm, absorption wavelength: 525 nm) were captured using a fluorescence microscope. Further, the obtained fluorescent image was analyzed by Hybrid Cell Count of Software (BZ-X Analyzer) attached to the fluorescence microscope. The extraction threshold for fluorescence was set to 40, and an integrated value of the luminance of each fluorescent image was calculated. The CS-TA-conjugate (Example 1) was used as the positive control compound for each test, and a relative value when using this as a criterion was calculated.

(4) Evaluation Result

A bright field image (a) of the frozen section on day 1 after administering the test substance, and a fluorescent image (b) corresponding thereto are shown in FIGS. 1 to 6 (respective Examples) and FIGS. 7 to 9 (respective Comparative Examples). Note that in the fluorescent image (b), a white portion shows a portion emitting fluorescence.

As shown in FIGS. 1 to 6, in the chondroitin sulfate derivative administration groups (Examples), strong fluorescence was observed in a wide range in a transitional epithelium and a submucosal tissue of the bladder, which indicated that the permeability to the submucosal tissue of the bladder is high. On the other hand, in the chondroitin sulfate administration group in FIG. 7 and the hyaluronic acid derivative administration groups in FIGS. 8 to 9 (Comparative Examples), fluorescence in a transitional epithelium and a submucosal tissue of the bladder was weak, which indicated that the permeability to the submucosal tissue of the bladder is low.

Further, the integrated value of the luminance on day 1 after administering each test substance (a relative value based on the value of Example 1) is shown in Table 2.

[Table 2]

| | Compound | Integrated value of luminance (on day 1 after administration) |
|---|---|---|
| Example 1 | Fluorescently labeled CS-TA-conjugate | 1.00 |
| Example 2 | Fluorescently labeled CS-HYD-conjugate | 1.50 |

(continued)

| | Compound | Integrated value of luminance (on day 1 after administration) |
|---|---|---|
| Example 3 | Fluorescently labeled CS-NAP-conjugate | 1.83 |
| Example 4 | Fluorescently labeled CS-DEH-conjugate | 1.27 |
| Example 5 | Fluorescently labeled CS-DIC-conjugate | 2.36 |
| Example 6 | Fluorescently labeled CS-PRO-conjugate | 1.22 |
| Comparative Example 1 | Fluorescently labeled CS | 0.32 |
| Comparative Example 2 | Fluorescently labeled HA-TA-conjugate | 0.07 |
| Comparative Example 3 | Fluorescently labeled HA-CIN-conjugate | 0.05 |

As shown in Table 2, the fluorescently labeled compounds (chondroitin sulfate derivatives) of Examples 1 to 6 had a larger integrated value of the luminance on day 1 after administration than the fluorescently labeled compound (chondroitin sulfate) of Comparative Example 1 and the fluorescently labeled compounds (hyaluronic acid derivatives) of Comparative Examples 2 to 3. From the results, it can be said that the fluorescently labeled compounds (chondroitin sulfate derivatives) of Examples 1 to 6 can increase the permeability of the pharmaceutical active ingredient (API) into a submucosal tissue of the bladder.

<Evaluation Using Rat 2>

1. Comparison between Chondroitin Sulfate Derivative and Chondroitin Sulfate

According to the method described in the above-mentioned evaluation 1, after administering each of the fluorescently labeled compound (chondroitin sulfate derivative) of Example 1 and the fluorescently labeled compound (chondroitin sulfate) of Comparative Example 1, a fluorescent image on day 7 was measured, and an integrated value of the luminance was calculated. The results are shown in Table 3.

[Table 3]

| | Compound | Integrated value of luminance (on day 7 after administration) |
|---|---|---|
| Example 1 | Fluorescently labeled CS-TA-conjugate | 1.00 |
| Comparative Example 1 | Fluorescently labeled CS | 0.03 |

As shown in Table 3, the fluorescently labeled compound (chondroitin sulfate derivative) of Example 1 had a significantly larger integrated value of the luminance on day 7 after administration than the fluorescently labeled compound (chondroitin sulfate) of Comparative Example 1. From the results, it can be said that the chondroitin sulfate derivative is present in a submucosal tissue of the bladder in a larger amount than chondroitin sulfate.

2. Comparison between Chondroitin Sulfate Derivative and Pharmaceutical Active Ingredient (Single Agent)

(1) Preparation of Test Substance

A test substance was prepared by dissolving the chondroitin sulfate derivative (CS-TA-conjugate) of Example 1 in a 5 mM phosphate buffer solution containing 2.3% (v/v) glycerin. Further, a commercially available triamcinolone acetonide preparation (Kenacort) was used as a comparative compound (single agent). In both Example and Comparative Example, the dose of TA was set to 0.8 mg/animal.

(2) Pretreatment of Test Animal

The pretreatment was performed in the same manner as in the evaluation 1.

(3) Evaluation Method

One day after the pretreatment, 0.2 mL of each test substance was transurethrally administered into the bladder using a catheter (PE50) under isoflurane inhalation anesthesia and allowed to stay there for 30 minutes.

Seven days after administering the test substance, the animal was laparotomized under isoflurane inhalation anesthesia and exsanguinated and euthanized by cutting the abdominal aorta, and then, the bladder was collected. The bladder was homogenized in a mixed solution of methanol/buffer solution (10 mmol/L ammonium formate buffer solution (pH 6.0) : methanol = 3:2 (v/v)), and a measurement sample was prepared so that the tissue concentration was 0.05 g/mL.

Subsequently, the amount of TA in the measurement sample was quantitatively determined by an LC/MS/MS analysis under the following conditions using triamcinolone-6-$d_1$ acetonide-$d_6$ as an internal standard substance.

- Measurement apparatus

  HPLC: Nexera X2 (Shimadzu Corporation)
  MS: QTRAP 6500 (AB SCIEX)

- HPLC conditions

  Analysis column: reverse-phase column (Inertsil ODS, 2.1 mm × 150 mm, GL Science, Inc.)
  Analysis time: 7 min
  Flow rate: 0.4 mL/min
  Mobile phase: mobile phase A: a 0.1% (v/v) aqueous formic acid solution, mobile phase B: methanol
  Gradient
  Temperature: 40°C

- MS conditions

  Ionization mode: ESI (Turbo Spray Ion Drive)
  Measurement mode: MRM
  Detection mode: Positive

(4) Evaluation Results

The results of the concentration of API (TA) on day 7 after administering the test substance are shown in Table 4.

[Table 4]

| Compound | Concentration of API (TA) (ng/g) |
|---|---|
| CS-TA-conjugate | 20.73 |
| TA | 4.09 |

As shown in Table 4, in the case of the CS-TA-conjugate, the concentration of TA on day 7 after administration was higher than in the case of TA. That is, in the group in which the chondroitin sulfate derivative (CS-TA-conjugate) was administered, the pharmaceutical active ingredient (TA) was present in a larger amount than in the group in which

the pharmaceutical active ingredient (TA) single agent was administered.

From the above results, it can be said that the chondroitin sulfate derivative into which API was introduced can effectively deliver API to a submucosal tissue of the bladder as compared with the case of the API single agent.

As found from the above results, according to the present invention, a system that enables effective delivery of a pharmaceutical active ingredient (API) administered to the lumen of the bladder to a submucosal tissue can be provided.

The present application claims priority based on Japanese Patent Application No. 2019-183082 filed with the Japan Patent Office on October 3, 2019, the contents of which are incorporated herein by reference in their entirety.

## Claims

**1.** A transmucosal delivery system for a pharmaceutical active ingredient to a submucosal tissue of a bladder, comprising:

a conjugate of chondroitin sulfate and a hydrophobic compound, wherein
the hydrophobic compound contains the pharmaceutical active ingredient.

**2.** The delivery system according to claim 1, wherein the hydrophobic compound has a ClogP of 1 or more.

**3.** The delivery system according to claim 1 or 2, wherein the pharmaceutical active ingredient is released from the conjugate after being administered to a lumen of the bladder.

**4.** The delivery system according to any one of claims 1 to 3, wherein the release rate of the pharmaceutical active ingredient from the conjugate in a 10 mM phosphate buffer solution (pH 7.4) at 36°C is from 0.1 %/day to 5 %/day.

**5.** The delivery system according to any one of claims 1 to 4, wherein the hydrophobic compound is a compound in which the pharmaceutical active ingredient and a spacer forming molecule are covalently bonded to each other, and the spacer forming molecule is covalently bonded to the chondroitin sulfate.

**6.** The delivery system according to claim 5, wherein the spacer forming molecule is covalently bonded to the chondroitin sulfate through an amide bond.

**7.** The delivery system according to claim 5 or 6, wherein the pharmaceutical active ingredient is covalently bonded to the spacer forming molecule through an ester bond.

**8.** The delivery system according to any one of claims 5 to 7, wherein the spacer forming molecule is represented by the following general formula (I):

$$H_2N\text{-}(CH_2)_p\text{-}X \qquad (I)$$

(wherein p represents an integer of 1 to 12, each $-CH_2-$ may independently have a substituent; and X represents a hydroxy group, a carboxy group, or an amino group).

**9.** The delivery system according to any one of claims 1 to 8, wherein a weight average molecular weight of the chondroitin sulfate is 10 times or more the molecular weight of the pharmaceutical active ingredient.

**10.** The delivery system according to any one of claims 1 to 9, wherein the weight average molecular weight of the chondroitin sulfate is 4,000 or more.

**11.** The delivery system according to any one of claims 1 to 10, wherein the molecular weight of the pharmaceutical active ingredient is 600 or less.

**12.** A method for screening a drug candidate compound for treating a bladder disease, comprising:

a step of providing a compound;
a step of administering the compound to the lumen of the bladder; and
a step of analyzing the presence of the compound in a submucosal tissue of the bladder, wherein

the presence of the compound in the submucosal tissue of the bladder is used as an index of the drug candidate compound.

**13.** The method according to claim 12, wherein the presence of the compound is analyzed by imaging, mass spectrometry, an immunoassay, or radioactivity measurement.

**14.** The method according to claim 12, wherein the presence of the compound is analyzed by fluorescent imaging.

**15.** The method according to any one of claims 12 to 14, wherein the presence of the compound is analyzed 6 hours or more after the compound is administered to the lumen of the bladder.

**16.** A drug candidate compound screened by the method according to any one of claims 12 to 15.

**17.** A method for producing a drug for treating a bladder disease comprising the method according to any one of claims 12 to 15.

**18.** A method for giving drug efficacy to a submucosal tissue of the bladder, comprising:

a step of administering a conjugate of chondroitin sulfate and a hydrophobic compound to the lumen of the bladder of a subject, wherein
the hydrophobic compound contains a pharmaceutical active ingredient.

**19.** A method for treating a bladder disease, comprising a step of administering the delivery system according to any one of claims 1 to 11 to the lumen of the bladder of a subject.

**20.** A method for treating a bladder disease, comprising the method according to claim 18.

**21.** A method for transmucosally delivering a pharmaceutical active ingredient to a submucosal tissue of the bladder, comprising:

a step of administering a conjugate of chondroitin sulfate and a hydrophobic compound to the lumen of the bladder of a subject, wherein
the hydrophobic compound contains the pharmaceutical active ingredient.

**22.** A conjugate for use in transmucosal delivery of a pharmaceutical active ingredient to a submucosal tissue of the bladder, wherein

the conjugate is a conjugate of chondroitin sulfate and a hydrophobic compound, and
the hydrophobic compound contains the pharmaceutical active ingredient.

**23.** A method for transmucosally delivering a pharmaceutical active ingredient to a submucosal tissue of the bladder and releasing the pharmaceutical active ingredient in a sustained manner in the submucosal tissue by binding a hydrophobic compound containing the pharmaceutical active ingredient to chondroitin sulfate, comprising:
a step of administering a conjugate of the chondroitin sulfate and the hydrophobic compound to the lumen of the bladder of a subject.

**24.** A prodrug of a pharmaceutical active ingredient, wherein

the prodrug is a conjugate of chondroitin sulfate and a hydrophobic compound,
the hydrophobic compound releasably contains the pharmaceutical active ingredient, and
the prodrug is used for transmucosally delivering the pharmaceutical active ingredient to a submucosal tissue of the bladder.

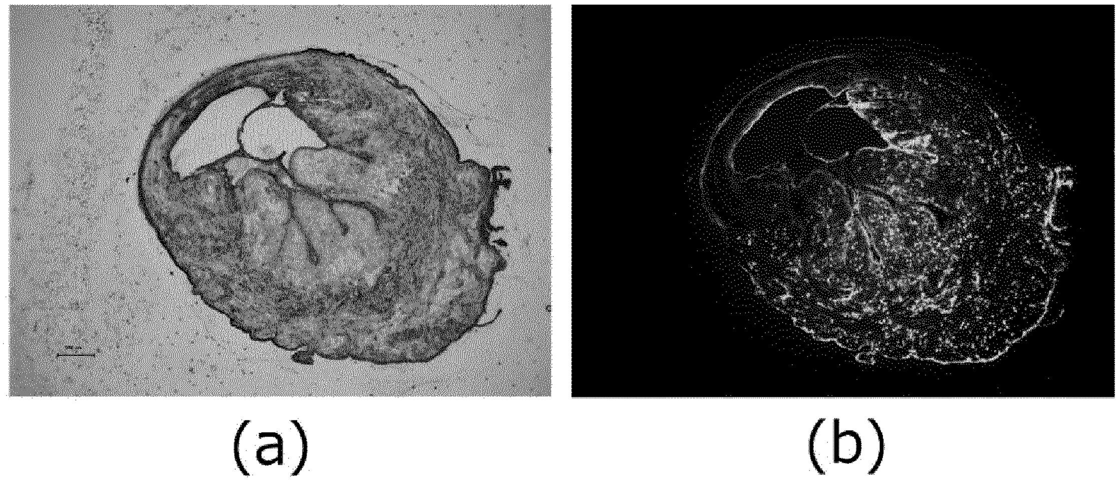


FIG. 1

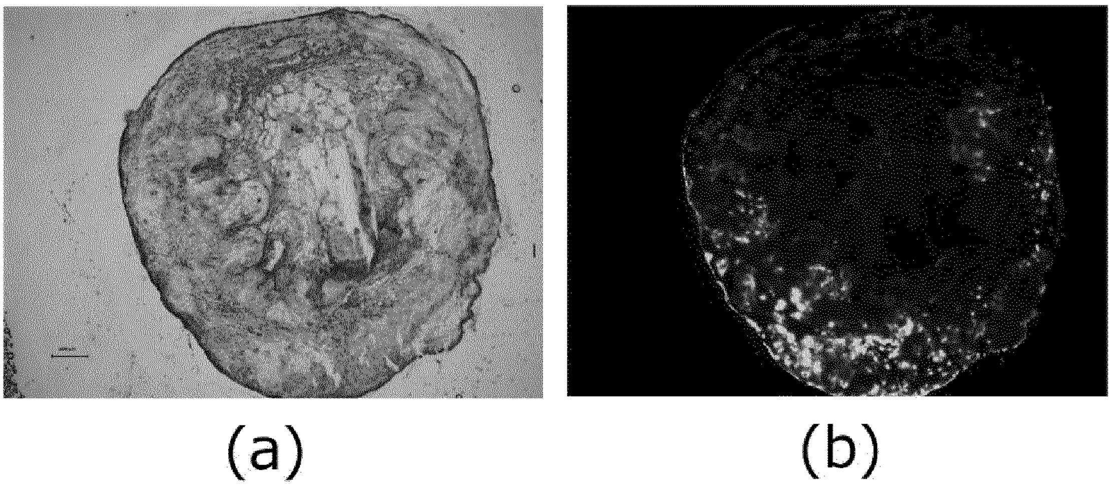


FIG. 2

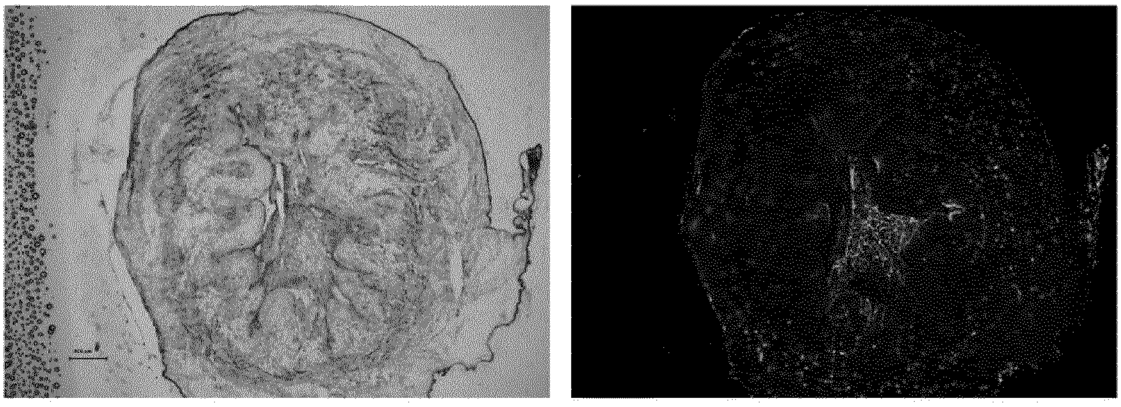

(a) (b)

FIG. 3

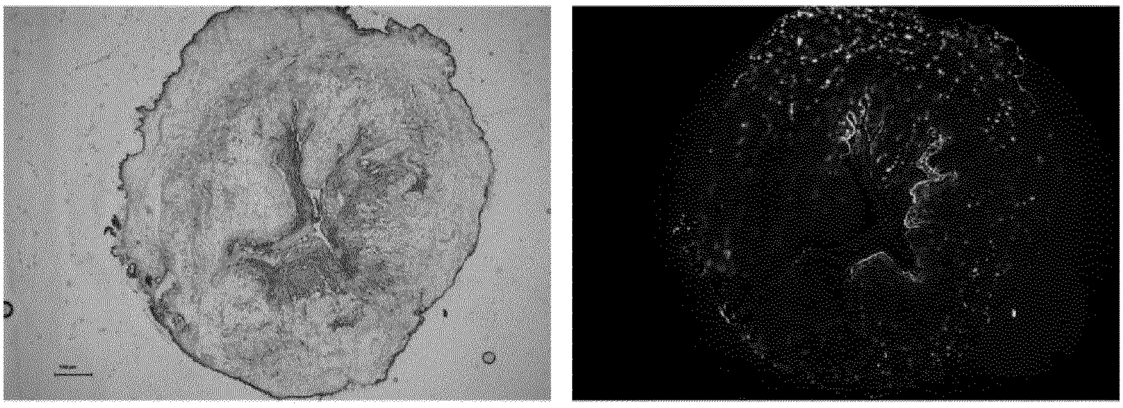

(a) (b)

FIG. 4

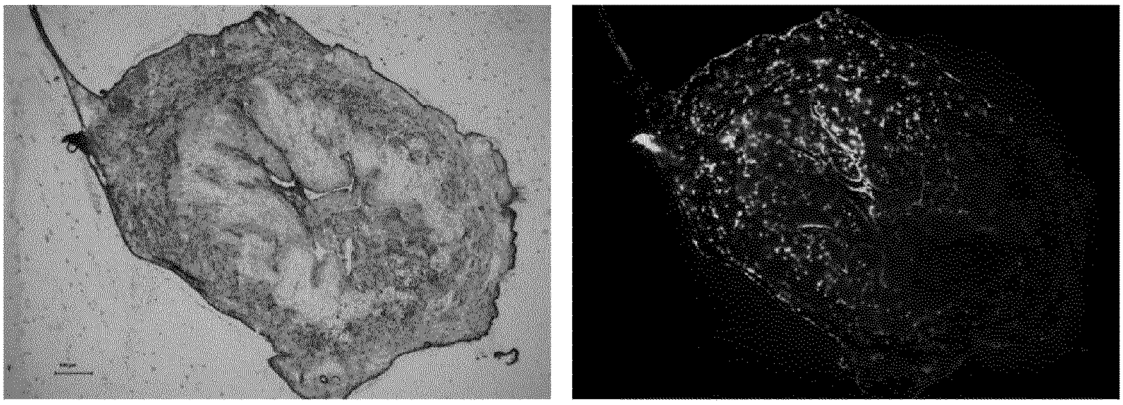

(a) (b)

FIG. 5

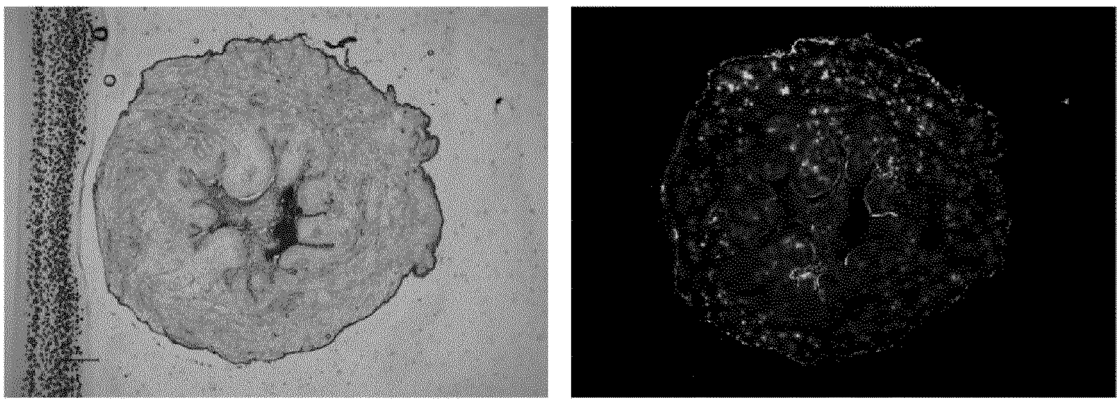

(a) (b)

FIG. 6

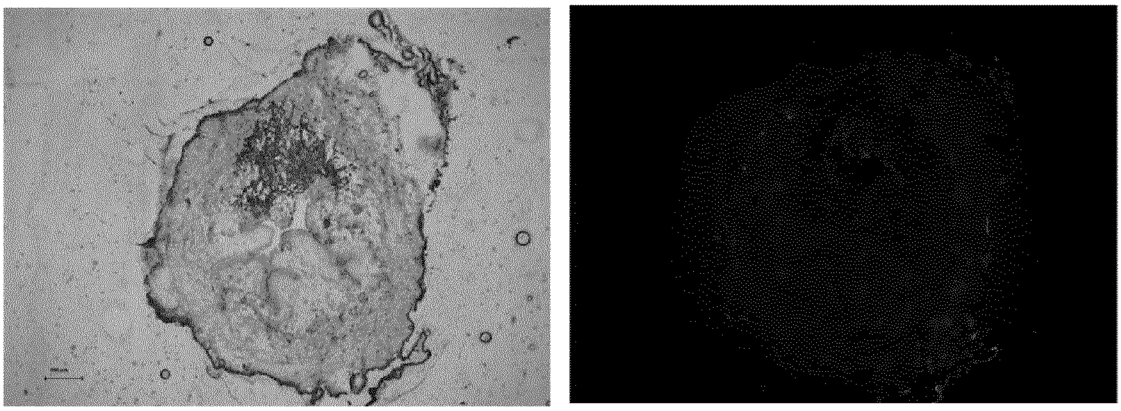

(a) (b)

FIG. 7

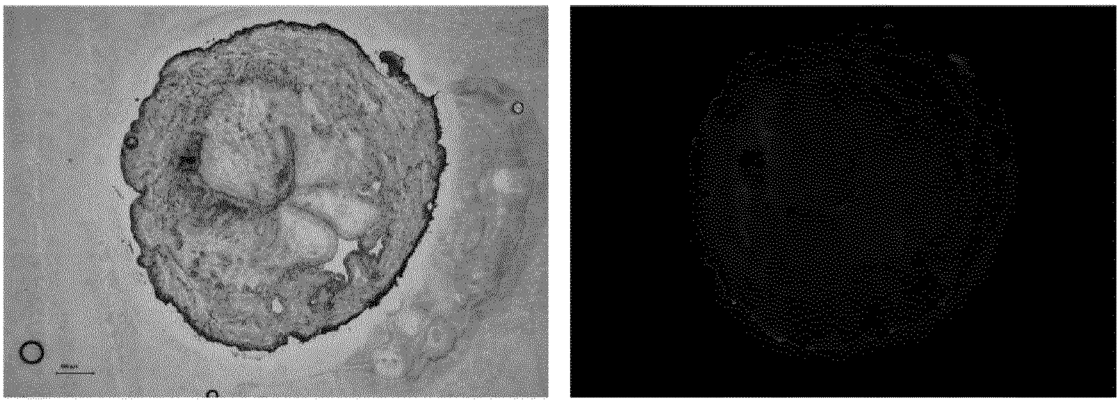

(a) (b)

FIG. 8

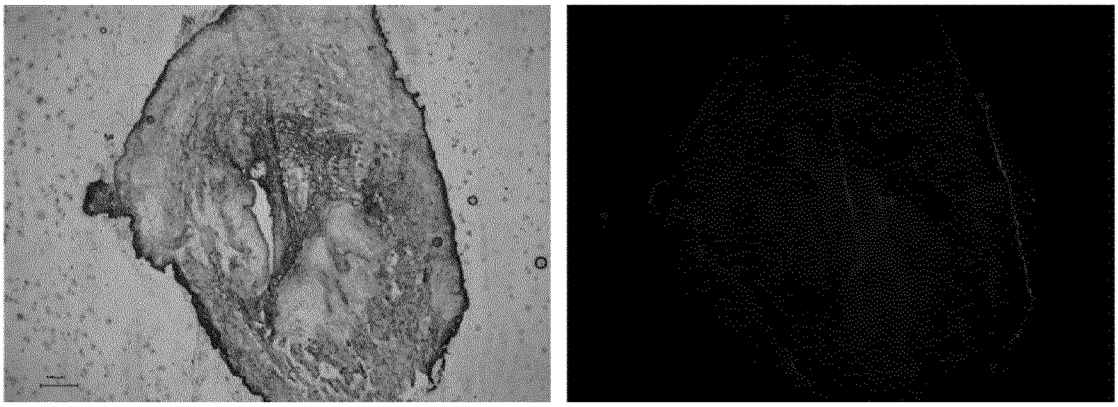

(a) (b)

FIG. 9

**INTERNATIONAL SEARCH REPORT**

International application No.
PCT/JP2020/037640

A. CLASSIFICATION OF SUBJECT MATTER
A61K 47/61(2006.01)i; A61P 13/10(2006.01)i; A61P 43/00(2006.01)i; A61K 45/00(2017.01)i; G01N 33/15(2006.01)i; G01N 33/50(2006.01)i; A61K 31/192(2006.01)i; A61K 31/196(2006.01)i; A61K 31/495(2006.01)i; A61K 31/575(2006.01)i; A61K 31/58(2006.01)i
FI: A61K47/61; A61P13/10; A61K45/00; A61P43/00 123; G01N33/50 Z; G01N33/15 Z; A61K31/58; A61K31/495; A61K31/575; A61K31/192; A61K31/196
According to International Patent Classification (IPC) or to both national classification and IPC

B. FIELDS SEARCHED
Minimum documentation searched (classification system followed by classification symbols)
A61K45/00; A61P13/10; A61P43/00; A61K47/61; G01N33/15; G01N33/50; A61K31/192; A61K31/196; A61K31/495; A61K31/575; A61K31/58

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| A | JP 2017-508749 A (TARIS BIOMEDICAL LLC) 30 March 2017 (2017-03-30) claims 1, 11, 13, 16, paragraphs [0015], [0051], fig. 14 | 1-11, 18-24 |
| X<br>Y | JP 2017-36455 A (SEIKAGAKU CORPORATION) 16 February 2017 (2017-02-16) claims, paragraphs [0005], [0006], [0009], [0014], [0017], [0020], [0021], [0026], [0027], [0031], [0038], [0039], [0057]-[0071], [0078]-[0083], [0087]-[0110], tables 3-6, fig. 1-4 | 1-11, 18-24<br>1-11, 18-21, 23 |
| A | 宮本葵ほか，液体クロマトグラフィー／質量分析法を用いる環境水中副腎皮質ステロイド類分析法の開発，BUNSEKI KAGAKU, 2010, vol. 59, no. 4, pp. 301-309 fig. 1, (MIYAMOTO, Aoi et al., "Determination of Corticosteroids in Environmental Water by Liquid Chromatography/Mass Spectrometry") | 1-11, 18-24 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>18 December 2020 (18.12.2020) | Date of mailing of the international search report<br>28 December 2020 (28.12.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/037640

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2015/005458 A1 (SEIKAGAKU CORPORATION) 15 January 2015 (2015-01-15) claims, examples 1-20, 36 | 22, 24<br>1-11, 18-21, 23 |
| X<br>Y | ONISHI, H. et al., "Conjugate between Chondroitin Sulfate and Prednisolone with Glycine Linker: Preparation and in Vitro Conversion Analysis", Chem. Pharm. Bull., 2013, vol. 61, no. 9, pp. 902-912 abstract, fig. 1, table 1 | 22, 24<br>1-11, 18-21, 23 |

Form PCT/ISA/210 (continuation of second sheet) (January 2015)

**INTERNATIONAL SEARCH REPORT**

International application No.
PCT/JP2020/037640

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-11, 18-24

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/JP2020/037640

<Continuation of Box No. III>

Document 1: JP 2017-508749 A (TARIS BIOMEDICAL LLC) 30 March 2017 (2017-03-30) claims 1, 11, 13, 16, paragraphs [0015], [0051], fig. 14 & US 2015/0250717 Al, claims, example 1

Claims are classified into the two inventions below.

(Invention 1) Claims 1-11, and 18-24

Claim 1 has the special technical feature of a "transmucosal delivery system of an active pharmaceutical ingredient to a submucosal tissue of a bladder, the delivery system including a combination with chondroitin sulfate and a hydrophobic compound, wherein the hydrophobic compound includes the active pharmaceutical ingredient." In addition, claims 2-11 and 18-24 have a special technical feature identical or corresponding to said special technical feature.

Therefore, claims 1-11, and 18-24 are classified as invention 1.

(Invention 2) Claims 12-17

Claims 12-17 and claim 1 classified as invention 1 share the common technical feature of delivering a compound to a submucosal tissue of a bladder. However, since the common technical feature does not make a contribution over the prior art in light of document 1 which indicates that a gemcitabine concentration is observed in the epithelium, lamina propria, muscularis, and adventitia of a bladder by locally administering gemcitabine into the bladder (claims 1, 11, 13, 16, paragraphs [0015], [0051], and fig. 14), the common technical feature cannot be a special technical feature. In addition, there are no other identical or corresponding special technical features between claims 12-17 and claim 1.

Further, claims 12-17 are not dependent on claim 1, and are not substantially identical or equivalent to any of the claims classified as invention 1.

Therefore, claims 12-17 cannot be classified as invention 1.

In addition, claims 12-17 have the special technical feature of a "method for screening drug candidate compounds for treating a bladder disease, the method comprising the steps of: providing a compound; administering the compound into the lumen of bladder; and analyzing the presence of the compound in the submucosal tissue of the bladder, wherein the presence of the compound in the submucosal tissue of the bladder is considered as an index of drug candidate compounds," and thus are classified as invention 2.

Form PCT/ISA/210 (extra sheet) (January 2015)

**INTERNATIONAL SEARCH REPORT**
Information on patent family members

International application No.
PCT/JP2020/037640

| Patent Documents referred in the Report | Publication Date | Patent Family | Publication Date |
|---|---|---|---|
| JP 2017-508749 A | 30 Mar. 2017 | US 2015/0250717 A1<br>claims, example 1 | |
| JP 2017-36455 A | 16 Feb. 2017 | US 2017 /0354675 A1<br>paragraphs [0010]-[0012], [0033], [0044], [0048], [0052]-[0053], [0060], [0064], [0082]-[0091], [0123]-[0153], [0166]-[0184], [0188]-[0252], tables 3-6, fig. 1-4<br>EP 3243841 A1<br>CN 107108761 A<br>KR 10-2017-0098942 A | |
| WO 2015/005458 A1 | 15 Jan. 2015 | US 2016/0151506 A1<br>claims 1-14, examples 1-20, 36<br>EP 3023108 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016111356 A
- WO 2007043702 A
- JP 2019183082 A

### Non-patent literature cited in the description


- **PAUL J. HAUSER.** Restoring Barrier Function to Acid-Damaged Bladder by Intravesical Chondroitin Sulfate. *The Journal of Urology,* 2009, vol. 182, 2477-2482
- **A. LEO.** Comprehensive Medicinal Chemistry. Pergamon Press, 1990, vol. 4, 315
- **LEO A. J.** *Chemical Reviews,* 1993, vol. 93, 1281-1306
- Chem Draw Professional. PerkinElmer, Inc,